# EUROPEAN PATENT APPLICATION

(11) **EP 3 835 432 A1**
(43) Date of publication of application: **16.06.2021**
(21) Application number: 19214890.6
(22) Date of filing: 10.12.2019
(51) Int. Cl.: C12Q 1/6886

(54) **INTERLEUKIN-4-INDUCED GENE 1 (IL4I1) AND RESPECTIVE METABOLITES AS BIOMARKERS FOR CANCER**

(71) Applicant: Deutsches Krebsforschungszentrum, Stiftung des öffentlichen Rechts, 69120 Heidelberg (DE); Helmholtz-Zentrum für Umweltforschung GmbH-UFZ, 04318 Leipzig (DE)
(72) Inventor: The designation of the inventor has not yet been filed
(74) Representative: Krauss, Jan

(57) **Abstract**

The present invention relates to the use of Interleukin-4-induced gene 1 (IL4I1) as well as metabolites as produced by IL4I1 as marker in diagnosis and therapy of cancer and related metastasis and/or resistance to immunotherapy.

## Description

The present invention relates to the use of Interleukin-4-induced gene 1 (IL4I1) as well as metabolites as produced by IL4I1 as markers in diagnosis and therapy of cancer and related metastasis and/or resistance to immunotherapy.

### Background of the invention

IL4I1 is an L-amino acid oxidase that catalyzes the oxidative deamination of L-amino acids to alpha-keto acids while producing hydrogen peroxide and ammonia. Initially discovered as an immediate-early IL4-inducible gene in B cells, IL4I1 was later on also identified in macrophages and dendritic cells (Molinier-Frenkel et al., 2019).

In cancer patients, IL4I1 is expressed either by tumor cells themselves (e.g., some B cell lymphoma subsets, mesothelioma or ovarian cancer and gliomas) or by tumor-associated macrophages (TAM) or dendritic cells (DC) (Carbonnelle-Puscian, et al. 2009). It is a secreted enzyme physiologically produced by antigen presenting cells (APC) of the myeloid and B cell lineages and T helper type (Th) 17 cells (Molinier-Frenkel et al., 2019). Important roles of IL4I1 in the fine control of the adaptive immune response in mice and humans have emerged during the last few years. Indeed, IL4I1 inhibits T cell proliferation (Lasoudris et al., 2011) and cytokine production and facilitates naive CD4+ T-cell differentiation into regulatory T cells *in vitro* by limiting the capacity of T lymphocytes to respond to clonal receptor stimulation. It may also play a role in controlling the germinal center reaction for antibody production and limiting Th1 and Th17 responses (Molinier-Frenkel et al., 2019).

Currently, only very little is known about the therapeutic implications of IL4I1-associated conditions. IL4I1 is expressed in tumor- associated macrophages of most human cancers and in some tumor cell types. Such expression, associated with its capacity to facilitate tumor growth by inhibiting the anti-tumor T-cell response, makes IL4I1 a new potential target in the field of immunomodulation in cancer.

Some compounds with alleged IL4I1 inhibitory activity were disclosed in WO 2010/066858. However, the specifically disclosed compounds were found either toxic or with unsatisfactory IL4I1 inhibitory activity. The disclosed compounds had Ki in the mM range, thus lacking efficacy, and rapidly induced complete cell death of a T cell line (Jurkat cells).

WO 2016/040488 discloses methods of promoting myelin formation in central nervous system (CNS) tissue in a subject in need thereof, the method comprising administering to the subject a therapeutically effective amount of IL4I1 protein.

WO 2019/185907 relates to the inhibition of IL4I1, found to be expressed in a large set of human cancers. Provided are compounds for a use in the treatment of cancer that are phenylalanine-derivatives according to a given formula (I). The cancer to be treated displays IL4I1-expressing cells.

There is also very limited information about diagnostic uses of IL4I1 and its metabolic functions in the context of cancer, in particular metastatic cancer.

Lasoudris et al. (in: IL4I1: an inhibitor of the CD8+ antitumor T-cell response in vivo, Eur J Immunol. 2011 Jun;41(6):1629-38) disclose that the L-phenylalanine oxidase IL4I1 inhibits T-cell proliferation in vitro through hydrogen peroxide (H₂O₂) production, and is highly expressed in tumor-associated macrophages. Immunosuppressive functions of IL4I1 are demonstrated in vivo and suggest that IL4I1 facilitates human tumor growth by inhibiting the CD8(+) antitumor T-cell response.

IL4I1 has also been disclosed as part of a so-called stroma-derived prognostic predictor (SDPP) that stratifies disease outcome in breast cancer, renal cancer, and glioma (Finak et al., Stromal gene expression predicts clinical outcome in breast cancer. Nat. Med. 2008;14:518-527).

Molinier-Frenkel et al. (The IL4I1 Enzyme: A New Player in the Immunosuppressive Tumor Microenvironment. Cells. 2019 Jul 20;8(7)) disclose that as a secreted enzyme, IL4I1 may represent an easily targetable molecule for cancer immunotherapy.

Bod L., et al. (IL4-induced gene 1 promotes tumor growth by shaping the immune microenvironment in melanoma. Oncoimmunology. 2017;6:e1278331) showed that IL4I1 activity correlated with disease aggressiveness, although they found that IL4I1 did not enhance tumor cell proliferation or angiogenesis.

Cheong and Sun (Targeting the IDO1/TDO2-KYN-AhR Pathway for Cancer Immunotherapy - Challenges and Opportunities. Trends Pharmacol Sci. 2018 Mar;39(3):307-325) review recent progress and future perspectives in targeting the IDO1/TDO2-KYN-AhR signaling pathway for the development of novel cancer immunotherapies.

So far, the secreted IL4I1 (interleukin-4-induced 1) enzyme was disclosed as catabolizing L-phenylalanine, and to a lesser extent arginine to generate H₂O₂, ammonia (NH₃) and the corresponding α-keto acid (Molinier-Frenkel et al., 2019; Boulland et al., 2007; Mason et al., 2004). However, it was found that IL4I1 is the key enzyme of a newly identified Trp-catabolic pathway that yields AHR agonists, including indole metabolites. IL4I1 associates with reduced survival in glioma patients, promotes cancer cell motility, and inhibits T-cell proliferation. Being a more potent AHR activator than IDOl, IL4I1 may explain the failure of clinical studies combining immune checkpoint blockade (ICB) with IDOl inhibitors: ICB induces IL4I1, which mediates therapy escape. Thus, IL4I1 blockade will open new avenues for cancer therapy.

An object of the invention is therefore to identify new approaches to use IL4I1 and its metabolic functions in methods for diagnosing and monitoring cancer. Other objects and aspects will become apparent when studying the following more detailed description of the invention.

The aryl hydrocarbon receptor (AHR) is a ligand-activated transcription factor that enables cells to adapt to changing conditions by sensing compounds from the environment, diet, microbiome, and cellular metabolism (Rothhammer and Quintana, 2019). Initially discovered as the mediator of the toxic effects of the xenobiotic 2,3,7,8-tetrachlorodibenzo-p-dioxin (TCDD, dioxin), the AHR was subsequently shown to exert important roles in development, immunity, and cancer (Boitano et al., 2010; Fernandez-Salguero et al., 1995; Gutierrez-Vazquez and Quintana, 2018; Kiss et al., 2011; Murray et al., 2014; Nguyen and Bradfield, 2008; Rentas et al., 2016; Stockinger et al., 2014). Upon ligand binding the AHR translocates from the cytoplasm into the nucleus, where it heterodimerizes with the AHR nuclear translocator (ARNT) and induces transcription by binding to xenobiotic response elements (XRE) (Nguyen and Bradfield, 2008). AHR target genes regulate a variety of biological processes, including angiogenesis, hematopoiesis, drug and lipid metabolism, cell motility, and immune modulation (Denison and Nagy, 2003; Gutierrez-Vazquez and Quintana, 2018; Murray et al., 2014; Nguyen and Bradfield, 2008; Stockinger et al., 2014). Next to xenobiotics, natural ligands derived from plants, microbiota and endogenous metabolism are potent AHR agonists (Denison and Nagy, 2003; Li et al., 2011; Rothhammer and Quintana, 2019). Tryptophan (Trp) derivatives constitute an important class of endogenous AHR ligands (Denison and Nagy, 2003; Hubbard et al., 2015; Nguyen and Bradfield, 2008). The kynurenine (Kyn) pathway, initiated by indoleamine-2,3-dioxygenase 1/2 (IDO1/2) or tryptophan-2,3-dioxgenase (TDO2), is currently considered as the main Trp catabolic route in humans (Cervenka et al., 2017; Lemos et al., 2019; Platten et al., 2019), yielding the AHR agonists Kyn and kynurenic acid (KynA) (DiNatale et al., 2010; Mezrich et al., 2010; Opitz et al., 2011). Cancers express high levels of IDOl and TDO2, and take advantage of the effects of Trp catabolite-mediated AHR activation to enhance their malignancy and to inhibit anti-tumor immune responses.

The Kyn-AHR axis suppresses T cell proliferation and function by inducing (i) the differentiation of regulatory T cells (Tregs) (Mezrich et al., 2010; Quintana et al., 2010), (ii) the expression of the immune checkpoint molecule programmed cell death protein 1 (PD-1) on CD8⁺ T cells (Liu et al., 2018), (iii) cell death of CD8⁺ T cells (Greene et al., 2019), and (iv) the recruitment of immunosuppressive tumor-associated macrophages (TAM) (Takenaka et al., 2019). Next to immune effects, the Kyn-AHR axis also enhances the malignant phenotype of cancer cells, the most prominent of which is cancer cell motility (Chen et al., 2014; D'Amato et al., 2015; Novikov et al., 2016; Opitz et al., 2011; Xiang et al., 2019).

Inhibition of Trp-catabolizing enzymes (TCEs) thus represents a promising strategy to target both cancer cell malignancy and tumor-derived immunosuppression (Lemos et al., 2019; Platten et al., 2019).

Small molecule inhibitors of IDO1 have entered clinical trials as an adjunct to immune checkpoint blockade (ICB) to improve therapy outcome by concomitantly targeting these two immunosuppressive mechanisms (Lemos et al., 2019; Platten et al., 2019). However, the first phase III clinical trial failed (Long et al., 2019), challenging the inventors' knowledge of Trp catabolism in cancer (Muller et al., 2019; Platten et al., 2019). A molecular understanding of this disappointing result should reveal new opportunities for immunotherapy.

The inventors thus hypothesized that other pathways that activate the AHR could constitute a resistance mechanism against IDOl inhibitors. To identify new mediators of AHR activation in human tumors, a straightforward approach would be to assess the association of metabolic enzymes beyond IDO1 and TDO2 with the expression of AHR target genes. However, the context-specificity of AHR target gene expression (Rothhammer and Quintana, 2019), which varies greatly across different tissues and ligands, impedes pan-cancer analysis of AHR activity. To meet this challenge, the inventors developed an AHR signature that enables detection of AHR activity, irrespective of cell type or ligand. This pan-tissue AHR signature allows it to assess whether other Trp-degrading enzymes beyond IDO1 and TDO2 activate the AHR in human cancers.

The inventors identified interleukin-4 induced 1 (IL4I1) as a major AHR-activating enzyme in human cancer, which associates more frequently with AHR activity than IDOl or TDO2. IL4I1 enhances tumor cell migration and suppresses anti-tumor immunity. As the key enzyme of a novel Trp catabolic pathway in humans, IL4I1 activity yields indole metabolites and KynA that act as AHR agonists. ICB induces IL4I1, likely mediating resistance to IDOl inhibitors in combinatorial therapies. Thus, IL4I1 targeting constitutes a new strategy to relieve resistance to immunotherapy and opens new avenues for AHR research.

In a first aspect thereof, the present invention relates to a method for detecting and/or diagnosing cancer in a patient comprising detecting the expression and/or the enzymatic activity of IL4I1 in a sample obtained from said patient, and diagnosing and/or detecting cancer in said patient, if said expression or enzymatic activity of said IL4I1 is increased in said patient when compared to control genes e.g, one or more housekeeping genes in said sample, or a control sample, e.g. a sample derived from a healthy subject, a group of healthy subjects, a prior sample from the same patient, a different patient or patient group.

In a second aspect thereof, the present invention provides a method for detecting increased tumor cell motility and metastasis in a cancer patient comprising detecting the activity and/or expression of IL4I1 in a sample obtained from said patient, and detecting increased tumor cell motility and metastasis in said patient, if said expression or enzymatic activity of said IL4I1 is increased in said patient when compared to control genes e.g, one or more housekeeping genes in said sample, or a control sample, e.g. a sample derived from a healthy subject, a group of healthy subjects, a prior sample from the same patient, a different patient or patient group.

In a third aspect thereof, the present invention provides a method for predicting or detecting the effect of cancer immunotherapy in a cancer patient comprising detecting the activity and/or expression of IL4I1 in a sample obtained from said patient, wherein said expression or enzymatic activity of said IL4I1 is increased in said patient when compared to control genes e.g, one or more housekeeping genes in said sample, or a control sample, e.g. a sample derived from a healthy subject, a group of healthy subjects, a prior sample from the same patient, a different patient or patient group is predictive for and/or indicates a reduced effect and/or immune evasion of said cancer.

In a fourth aspect thereof, the present invention provides a method for detecting resistance against a cancer treatment comprising immunotherapy, such as immune checkpoint blockade (ICB), in a patient comprising detecting the activity and/or expression of IL4I1 in a sample obtained from said patient, and detecting resistance against a cancer treatment comprising ICB in said patient, if said expression or enzymatic activity of said IL4I1 is increased in said patient when compared to control genes e.g, one or more housekeeping genes in said sample, or a control sample, e.g. a sample derived from a healthy subject, a prior sample from the same patient, a different patient or patient group.

In a fifth aspect thereof, the present invention provides a method for predicting survival in a cancer patient comprising detecting the activity and/or expression of IL4I1 in a sample obtained from said patient, wherein said expression or enzymatic activity of said IL4I1 is increased in said patient when compared to control genes e.g, one or more housekeeping genes in said sample, or a control sample, e.g. a sample derived from a healthy subject, a group of healthy subjects, a prior sample from the same patient, a different patient or patient group, is predictive for a reduced survival in said patient.

Another important aspect of the present invention relates to a diagnostic kit comprising materials for performing a method according to the present invention in one or separate containers, optionally together with auxiliary agents and/or instructions for performing said method. Another important aspect of the present invention then relates to the use of said diagnostic kit in a method according to the present invention.

Generally preferred are the human variants of the biomarker IL4I1 and/or AHR, or closely related species, like the ones from other primates or mammals, such as rodents.

Other aspects and advantages can be readily derived from reading the following description and the non-limiting examples.

The present inventors - while investigating the role of tryptophan degrading enzymes in modulating AHR activity - discovered that the expression of IL4I1, a tryptophan-degrading enzyme expressed in human cancers and not yet implicated in AHR activation, correlated significantly with AHR target gene expression. Gene expression analyses and AHR nuclear translocation established IL4I1 to activate the AHR via the production of tryptophan metabolites including indole-3-pyruvic acid (I3P) and kynurenic acid (see Table 1).

Another important aspect of the present invention then relates to a method for detecting and/or diagnosing cancer in a patient comprising detecting the expression and/or enzymatic activity of IL4I1 in a sample obtained from said patient, and diagnosing and/or detecting cancer in said patient, if said expression or enzymatic activity of said IL4I1 is increased in said patient when compared to control genes e.g, one or more housekeeping genes in said sample, or a control sample, e.g. a sample derived from a healthy subject, a group of healthy subjects, patient or patient group.

In one aspect, said method comprises detecting the expression and/or enzymatic activity or biological function of IL4I1 in a cell/tissue/biological fluid, wherein a change in the expression, enzymatic activity or biological function in said compartments, in particular expression or activation, when compared to a healthy or other suitable control sample, indicates cancer. Such a change can be at least about 5%, at least about 10%, at least about 20%, at least about 30%, at least about 40%, at least about 50%, at least about 60%, at least about 70%, at least about 80% or at least about 90% or more of up or down regulation of expression or enzymatic activity or biological function, when compared with a suitable control, such as the value in a healthy cell or a sample derived from a healthy person or group of individuals, or when compared to an internal standard, like a housekeeping gene. In the context of the present invention, the term "about" shall mean +/- 5% of the given value, unless indicated otherwise.

In the context of the present invention, it was found that the modulation of downstream effects of IL4I1, such as AHR modulation, is caused by the metabolites that are the result of the enzymatic activity or biological function of IL4I1 in a cell/tissue. These metabolites are then, for example, also found in biological fluids, which allows for a more convenient detection of these oncometabolites. I3P and, in particular metabolites derived from I3P represent novel oncometabolites that mediate IL4I1 and AHR-driven malignant properties.

Preferably, the detection of said enzymatic activity or biological function of IL4I1 in said sample comprises the detection of the amount and/or concentration of IL4I1 metabolites in said sample. These metabolites are selected from metabolites derived from the conversion of IL4I1 of phenylalanine, tyrosine and/or tryptophan, such as, for example, phenylpyruvic acid (PP), hydroxyphenylpyruvic acid (HPP), indole-3-pyruvic acid (I3P), 2-phenylacetic acid, phenyllactic acid, 4-hydroxybenzaldehyde, 2-hydroxy-2-phenylacetic acid, 4-hydroxyphenyllactic acid, and in particular the I3P derivatives indole-3-acetic acid (IAA), indole-3-aldehyde (I3A) and indole-3-lactic acid (ILA), 4-hydroxyquinoline-2-carboxylic acid (KynA), 1,3-di(1H-indole-3-yl)acetone, (3*Z*)-1-(1*H*-indole-3-yl)-3-indole-3-ylidenepropan-2-one, indole-3-carboxylic acid, oxidized indole-3-acetic acid, and indole-3-carbinol and/or the amino acids or amino acid metabolites L-valine, L-isoleucine, L-leucine, L-alanine, L-glutamic acid, L-methionine, L-glutamine, 4-methylsulfanyl-2-oxobutanoate, alpha-keto-isoleucine, alpha-ketoisovalerate, alpha-ketoisocaproic acid, L-proline, and alpha-ketoglutaric acid, and any of the above in combination with ammonia and/or H₂O₂.

In the methods according to the present invention detecting the enzymatic activity or biological function of IL4I1, in particular the generation of metabolites as disclosed herein, can be performed in any suitable manner, preferred is the use of a peroxide cleavable substrate that can be detected.

Another aspect of the invention then relates to a method for detecting increased tumor cell motility in a cancer patient comprising detecting the enzymatic activity and/or expression of IL4I1 in a sample obtained from said patient, and detecting increased tumor cell motility in said patient, if said activity and/or expression of said IL4I1 is increased in said patient when compared to a control sample or a previous sample acquired from the same patient, or samples acquired from different patients or patient groups.

The inventors show here that IL4I1 is expressed not only in immune cells (Carbonnelle-Puscian et al., 2009), but also in cancer cells that do not derive from the immune system. For the first time, the inventors link IL4I1 to tumor intrinsic malignant properties, including cancer cell migration and metastasis.

Preferably, said cancer is characterized by a modulation, such as an increase of the activity and/or expression of AHR, and/or is selected from the group consisting of B cell lymphoid malignancies, such as follicular lymphoma, Hodgkin lymphoma, primary mediastinal B cell lymphoma, diffuse large B cell lymphoma, marginal zone lymphoma and chronic lymphoid leukemia, ovarian carcinomas, mesotheliomas, colon carcinomas, breast carcinomas, melanomas, glioblastomas prostate cancer, endometrial cancer, and lung carcinomas, preferably displaying IL4I1 -expressing cells, and relapsing and metastasizing forms thereof.

IL4I1 promotes tumor cell motility. To test IL4I1's contribution to this phenotype and the functional outcomes of the AHR, the inventors investigated the role of IL4I1 in GBM cell motility. Ectopic IL4I1 increased the motility of AHR-proficient cells, but not of AHR-deficient cells. In keeping with this result, the IL4I1-mediated motility of AHR-proficient cells was reversed by the AHR inhibitor SR1. The finding that IL4I1 acts through the AHR, with its major outputs in immunity and cancer cell migration, enabled the inventors to identify IL4I1 as a driver of cancer cell motility.

AHR-driven motility contributes to the malignant properties of gliomas and other cancer entities, but has so far mainly been linked to its upstream metabolic regulators IDO1 and TDO2. The inventors show here that IL4I1 is of greater importance in cancer such as glioma survival than IDO1 and TDO2, and this strong negative association with glioma survival is at least in part due to IL4I1's pro-migratory effects. IL4I1's migratory outcomes do not only explain its importance in glioma but also underlie its impact in metastatic cancers, such as melanoma. In this context, it is important to note that unlike IDOl and TDO2, IL4I1 is secreted. Thus, in addition to being detected more conveniently in assays, IL4I1 might enhance systemic Trp-I3P catabolism, thereby contributing to a systemic pro-metastatic environment that allows metastatic cells to migrate and protects them from immune destruction.

Preferably, said detecting of said activity and/or expression comprises detecting the activity and/or expression of AHR, wherein an increase in said activity and/or expression of AHR when compared to a control is indicative for an increase of the activity of IL4I1. The activity of IL4I1 can then, for example, be determined using the metabolites and test therefore as described above. Expression can be detected using genetic tools, such as chip analysis and primers and probes and PCR analysis, or detecting the amount of protein of IL4I1 using, for examples, antibodies, in samples, like biological fluids and cells/ tissue samples.

While the state of the art has speculated about a possible role of IL4I1 in cancer cell motility, these reports have been made in the context of large chip expression signatures, and also lack the important context of AHR and IL4I1, as well as the metabolic functions of IL4I1.

Another aspect of the invention then relates to a method for predicting or detecting the effect and/or suitability of cancer immunotherapy in a cancer patient, comprising detecting the activity and/or expression of IL4I1 in a sample obtained from said patient, wherein said expression or enzymatic activity of said IL4I1 is increased in said patient when compared to control genes e.g, one or more housekeeping genes in said sample, or a control sample, e.g. a sample derived from a healthy subject, a group of healthy subjects, a prior sample from the same patient, a different patient or patient group, is predictive for and/or indicates a reduced effect, suitability and/or immune evasion of said cancer immunotherapy for/in said patient.

Importantly, the inventors show that IL4I1 is expressed not only in immune cells (Carbonnelle-Puscian et al., 2009), but also in cancer cells that do not derive from the immune system. For the first time, the inventors link IL4I1 to tumor intrinsic malignant properties, including cancer immune evasion. The inventors show that catabolism of Trp by the L-amino-acid oxidase IL4I1 elicits major effects in immunity and cancer by signaling through the I3P-AHR axis. Until now IL4I1 has been merely implicated in immune regulatory functions that have been attributed either to the depletion of amino acids, the formation of H₂O₂ or an enzyme-independent function of IL4I1 (Molinier-Frenkel et al., 2019).

Thus, IL4I1 targeting constitutes a new strategy to relieve resistance to immunotherapy.

As mentioned above, the activity of IL4I1 can then, for example, be determined using the metabolites and test therefore as also described above. Expression can be detected using genetic tools, such as chip analysis and primers and probes and PCR analysis, or detecting the amount of protein of IL4I1 using, for examples, antibodies, in samples, like serum samples.

The term "immunotherapy" as used herein shall include any artificial stimulation of the immune system to treat cancer, such as cellular immunotherapy, cancer vaccination(s), anti-cancer antibody treatments, and cytokine treatments. Particularly mentioned shall be a cancer treatment comprising immune checkpoint blockade (ICB), such as, for example, comprising the use of IDOl inhibitors (see, for example, Prendergast GC, et al.. Discovery of IDOl Inhibitors: From Bench to Bedside. Cancer Res. 2017;77(24):6795-6811, and Cheong JE, et al. A patent review of IDOl inhibitors for cancer. Expert Opin Ther Pat. 2018 Apr;28(4):317-330), and/or antibodies selected from anti-CTLA-4, anti-PD-1, anti-PDL-1, anti LAG3 and anti BTLA, and adoptive immunotherapy.

Particularly preferred is a method according to the present invention, wherein said increase of said activity and/or expression of said IL4I1 is detected in response to an immunotherapy in said patient. In the context of the present invention, it was shown for the first time that immune evasion is actively linked to the increased activity and/or expression of IL4I1 and the IL4I1-AHR axis.

Another aspect of the invention then relates to a method for detecting resistance against a cancer treatment comprising immune therapy, for example immune checkpoint blockade (ICB), in a patient comprising detecting the activity and/or expression of IL4I1 in a sample obtained from said patient, and detecting resistance against a cancer treatment comprising immune therapy in said patient, if said expression or enzymatic activity of said IL4I1 is increased in said patient when compared to control genes e.g, one or more housekeeping genes in said sample, or a control sample, e.g. a sample derived from a healthy subject, a prior sample from the same patient, a different patient or patient group.

Preferably, said immune therapy treatments comprise at least one single immune therapy intervention, for example ICB treatment that comprises the use of IDO1 inhibitors (see, for example, Prendergast GC, et al.. Discovery of IDOl Inhibitors: From Bench to Bedside. Cancer Res. 2017;77(24):6795-6811, and Cheong JE, et al. A patent review of IDOl inhibitors for cancer. Expert Opin Ther Pat. 2018 Apr;28(4):317-330) and/or antibodies selected from anti-CTLA-4, anti-PD-1, anti-PDL-1, anti LAG3 and anti BTLA, used as a single line of treatment or in combination with one or more cancer therapeutic, for example kinase inhibitors, nuclear receptor antagonists or other chemotherapeutic agents

Similar to the aspect above, IL4I1 targeting constitutes a new strategy to relieve resistance to immunotherapy, in this case in the context of ICB-related cancer treatment. As mentioned above, the activity of IL4I1 can then, for example, be determined using the metabolites and test therefore as also described above. Expression can be detected using genetic tools, such as chip analysis and primers and probes and PCR analysis, or detecting the amount of protein of IL4I1 using, for examples, antibodies, in samples, like biological fluids or tissue samples. Particularly preferred is a method according to the present invention, wherein said increase of said activity and/or expression of said IL4I1 is detected in response to an ICB immunotherapy in said patient. In the context of the present invention, it was shown for the first time that immune evasion is actively linked to the increased activity and/or expression of IL4I1 and the IL4I1-AHR axis. The consequence of a positive finding based on this method would be the use of an IL4I1 inhibitor for therapy.

Another aspect of the invention then relates to a method for predicting survival in a cancer patient comprising detecting the activity and/or expression of IL4I1 in a sample obtained from said patient, wherein said expression or enzymatic activity of said IL4I1 is increased in said patient when compared to control genes e.g, one or more housekeeping genes in said sample, or a control sample, e.g. a sample derived from a healthy subject, a prior sample from the same patient, a different patient or patient group, is predictive for a reduced survival in said patient.

As mentioned above, the activity of IL4I1 can then, for example, be determined using the metabolites and test therefore as also described above. Expression can be detected using genetic tools, such as chip analysis and primers and probes and PCR analysis, or detecting the amount of protein of IL4I1 using, for examples, antibodies, in samples, like serum samples.

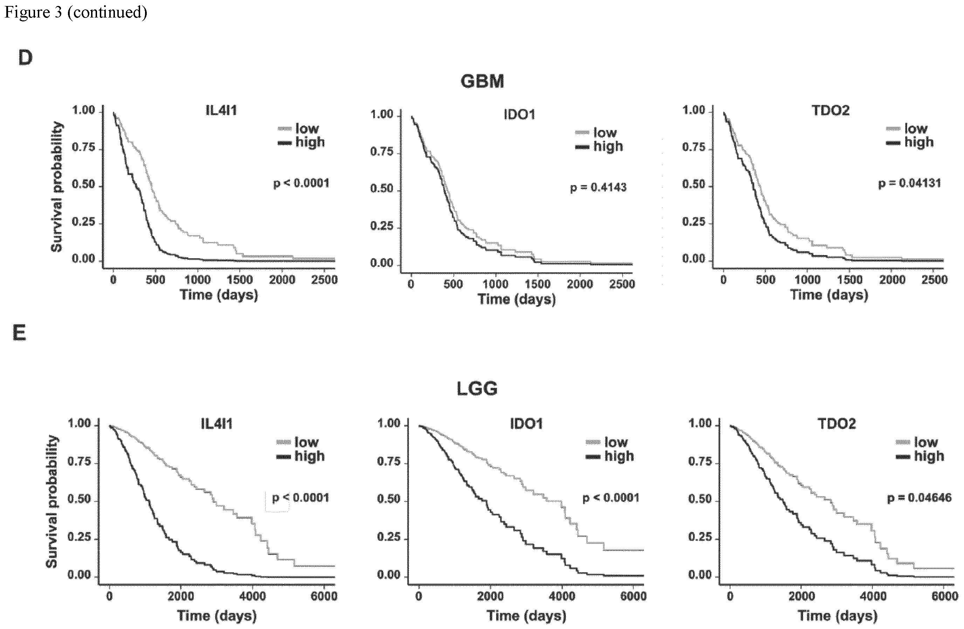

The inventors show here that IL4I1 and the IL4I1-AHR axis is of greater importance in cancer such as for glioma survival compared to IDO1 and TDO2, and this strong negative association with glioma survival is at least in part due to IL4I1's pro-migratory effects. As an example, the inventors analyzed the association of the three AHR-activating enzymes IL4I1, IDOl, and TDO2, with overall survival probability in GBM. High *IL4I1* levels associated with reduced overall survival (Figure 3D). In fact, GBM patients with high tumoral *IL4I1* expression had a 2.3 times higher risk of death compared to patients with low *IL4I1* expression (Figure 3D, and Table 2). High *TDO2* levels associated with a 1.5 times higher risk of death, while no significant survival difference was associated with *IDOl* transcript levels (Figure 3D and Table 2). Similarly, in low grade glioma (LGG) high expression of *IL4I1, IDO1* and *TDO2* were all associated with a higher risk of death (Figure 3E). The risk increase was highest for *IL4I1* expression (3.3 fold), followed by *IDOl* (2.4 fold), and *TDO2* (1.6 fold) (Figure 3E, and Table 2).

As also mentioned above, preferred is a method according to the present invention, wherein said detecting of said activity comprises detecting IL4I1 metabolites (oncometabolites), in particular IL4I1 tryptophan metabolites such as, for example I3P and/or the I3P derivatives indole-3-acetic acid (IAA), indole-3-aldehyde (I3A) and indole-3-lactic acid (ILA). I3P, and in particular the downstream metabolites thereof as disclosed herein, represent novel oncometabolites that mediate IL4I1 and AHR-driven malignant properties.

Preferably, the detection of said enzymatic activity or biological function of IL4I1 in said sample comprises the detection of the amount and/or concentration of IL4I1 metabolites in said sample. These metabolites are selected from metabolites derived from the conversion of IL4I1 of phenylalanine, tyrosine and/or tryptophan, such as, for example, phenylpyruvic acid (PP), hydroxyphenylpyruvic acid (HPP), indole-3-pyruvic acid (I3P), 2-phenylacetic acid, phenyllactic acid, 4-hydroxybenzaldehyde, 2-hydroxy-2-phenylacetic acid, 4-hydroxyphenyllactic acid, and in particular the I3P derivatives indole-3-acetic acid (IAA), indole-3-aldehyde (I3A) and indole-3-lactic acid (ILA), 4-hydroxyquinoline-2-carboxylic acid (KynA), 1,3-di(1H-indole-3-yl)acetone, (3Z)-1-(1*H*-indole-3-yl)-3-indole-3-ylidenepropan-2-one, indole-3-carboxylic acid, oxidized indole-3-acetic acid, and indole-3-carbinol and/or the amino acids or amino acid metabolites L-valine, L-isoleucine, L-leucine, L-alanine, L-glutamic acid, L-methionine, L-glutamine, 4-methylsulfanyl-2-oxobutanoate, alpha-keto-isoleucine, alpha-ketoisovalerate, alpha-ketoisocaproic acid, L-proline, and alpha-ketoglutaric acid, and any of the above in combination with ammonia and/or H₂O₂.

In the methods according to the present invention detecting the enzymatic activity or biological function of IL4I1 can be performed in any suitable manner, preferred is the use of chromatography, NMR, metabolite sensors, antibodies, ELISAs, enzymatic assays, colorimetric assays, fluorescence assays and/or H₂O₂ detection or ammonia detection.

Another aspect of the invention then relates to a method according to the present invention, wherein said detecting of said activity comprises detecting the activity and/or expression of AHR, wherein an increase in said activity and/or expression of AHR when compared to a suitable control is indicative for an increase of the activity of IL4I1. Detecting the activity and/or expression of AHR can be achieved in accordance with methods as disclosed in the state of the art, and the examples herein, and also can involve detection using genetic tools, such as chip analysis and primers and probes and PCR analysis, or detecting the amount of protein of IL4I1 using, for examples, antibodies, in samples, like biological fluids and cell/tissue samples.

In the context of the present invention, any biological sample comprising the marker protein IL4I1 (or functionally relevant parts thereof), or a sample comprising cells, comprising the marker protein IL4I1 (or functionally relevant parts thereof), e.g. obtained from a cancer patient, or a sample comprising at least one of the metabolites produced downstream of IL4I1, for example as shown in Table 1, can be used, as long as it contains (or is presumed to contain) at least one of the biomarker(s) (including AHR or AHR targets) or is presumed to have a perturbed AHR activity profile, to be used in the analysis.

Preferably, the biological sample is selected from a sample comprising biological fluids comprising biomarkers (in particular the present oncometabolites, AHR, and/or IL4I1), cells, a suitable sample comprising biological fluids, human cells, tissues, whole blood, cell lines, cellular supernatants, primary cells, IPSCs, hybridomas, recombinant cells, stem cells, and cancer cells, bone cells, cartilage cells, nerve cells, glial cells, epithelial cells, skin cells, scalp cells, lung cells, mucosal cells, muscle cells, skeletal muscles cells, striated muscle cells, smooth muscle cells, heart cells, secretory cells, adipose cells, blood cells, erythrocytes, basophils, eosinophils, monocytes, lymphocytes, T-cells, B-cells, neutrophils, NK cells, regulatory T-cells, dendritic cells, Th17 cells, Th1 cells, Th2 cells, myeloid cells, macrophages, monocyte derived stromal cells, bone marrow cells, spleen cells, thymus cells, pancreatic cells, oocytes, sperm, kidney cells, fibroblasts, intestinal cells, cells of the female or male reproductive tracts, prostate cells, bladder cells, eye cells, corneal cells, retinal cells, sensory cells, keratinocytes, hepatic cells, brain cells, kidney cells, and colon cells, and the transformed counterparts of said cells or tissues. The sample can also be selected from tumor tissue (tumor or metastases), biopsies, whole blood, peripheral blood, or fractions thereof, serum, buffy coat, lymphatic fluid, urine, bone marrow, heparinized whole blood, and frozen samples thereof, such as frozen heparinized whole blood. The cells to be used in the methods according to the present invention can be recombinant or non-recombinant, and express cell-foreign proteins, depending on the desired purpose and circumstances. Totipotent human embryonic stem cells may be excluded, if necessary. The sample can also be a combined sample from a group of subjects, for example, a group of healthy subjects, or a patient group.

In the methods according to the present invention the control sample can be selected from, for example, a sample from a healthy subject, an earlier (prior) sample from the same or a different similar patient, and/or from a group of subj ects/patients. Preferred is a method according to the present invention, wherein said subject is selected from a mammalian subject, in particular a human subject, in particular a human patient suffering from an IL4I1- and/or AHR-related physiological or pathological condition. Said control sample can be selected from a sample as described above.

Preferred is a method according to the present invention, wherein one or more reference genes could be used in determining the increase of the activity and/or expression of IL4I1.

Preferred is a method according to the present invention, wherein said cancer is characterized by a modulation, such as an increase or a decrease of the activity and/or expression of IL4I1 and/or AHR, and/or is preferably selected from the group consisting of B cell lymphoid malignancies, such as follicular lymphoma, Hodgkin lymphoma, primary mediastinal B cell lymphoma, diffuse large B cell lymphoma, marginal zone lymphoma and chronic lymphoid leukemia, ovarian carcinomas, mesotheliomas, colon carcinomas, breast carcinomas, melanomas, glioblastomas, prostate cancer, endometrial cancer, and lung carcinomas, preferably displaying IL4I1 -expressing cells, and any relapsing and metastasizing forms thereof.

Preferred is a method according to the present invention, further comprising the step of a stratification of said patient to a disease and/or treatment group, for example a treatment group receiving suitable IL4I1 inhibitors. Said stratification allows defining subgroups of patients with distinct mechanisms of disease, or particular responses to treatments in order to identify and develop treatments that are effective for particular groups of patients.

In another aspect of the present invention, the invention then relates to a diagnostic kit comprising materials for performing a method according to the present invention as herein in one or separate containers, optionally together with auxiliary agents and/or instructions for performing said methods according to the present invention.

Another aspect of the present invention then relates to the use of a diagnostic kit according to the present invention in a method according to the present invention.

In another preferred aspect of the present invention, the invention then relates to a method of treating and/or preventing an IL4I1-related disease or condition, in particular cancer, such as AHR-related cancer, in a cell in a patient in need of said treatment, comprising performing a method according to the present invention, and providing a suitable treatment to said patient, wherein said treatment is based, at least in part, on the results of the method according to the present invention, such as a stratification.

Particularly preferred is a method according to the present invention, wherein said treatment comprises an inhibition of the expression and/or enzymatic activity of IL4I1, more preferably leading to a reduction of IL4I1 expression or IL4I1 associated oncometabolites, possibly in combination with immunotherapy, and/or the co-inhibition of IDO1 or TDO2, to achieve efficient de-repression of anti-tumor immunity.

"Treatment" or "treating" shall mean a reduction and/or amelioration of the symptoms of the disease. An effective treatment achieves, for example, a shrinking of the mass of a tumor and the number of cancer cells. A treatment can also avoid (prevent) and reduce the spread of the cancer, such as, for example, affect metastases and/or the formation thereof. A treatment may be a naive treatment (before any other treatment of a disease had started), or a treatment after the first round of treatment (e.g. after surgery or after a relapse). The treatment can also be a combined treatment, involving, for example, chemotherapy, surgery, immunotherapy, small molecule inhibitors and/or radiation treatment.

In addition to the above, in the methods of the present invention, in general the IL4I1 and/or AHR can be detected and/or determined using any suitable assay. Detection is usually directed at the qualitative information ("marker yes-no"), whereas determining involves analysis of the quantity of a marker (e.g. expression level and/or activity). Detection is also directed at identifying for example mutations that cause altered functions of individual markers. The choice of the assay(s) depends on the parameter of the marker to be determined and/or the detection process. Thus, the determining and/or detecting can preferably comprise a method selected from subtractive hybridization, microarray analysis, DNA sequencing, RNA sequencing, qPCR, ELISA, IP, PLA, BiFC, HPLC, WB, enzymatic activity tests, fluorescence detection, cell viability assays, for example an MTT assay, phosphoreceptor tyrosine kinase assays, phospho arrays and proliferation assays, for example the BrdU assay, proteomics, cytokine arrays, and mass spectrometry.

Preferably, the inventive methods are also amenable to automation, and e.g. said activity and/or expression is preferably assessed in an automated and/or high-throughput format. Usually, this involves the use of chips and respective machinery, such as robots.

The inventors report that catabolism of Trp by the L-amino-acid oxidase IL4I1 elicits major effects in immunity and cancer by signaling through the I3P-AHR axis. Until now IL4I1 has been merely implicated in immune regulatory functions that have been attributed either to the depletion of amino acids, the formation of H₂O₂ or an enzyme-independent function of IL4I1 (Molinier-Frenkel et al., 2019).

The inventors show here that IL4I1 is expressed not only in immune cells (Carbonnelle-Puscian et al., 2009), but also in cancer cells that do not derive from the immune system. For the first time, the inventors link IL4I1 to tumor intrinsic malignant properties, including cancer cell migration and metastasis.

The finding that IL4I1 acts through the AHR, with its major outputs in immunity and cancer cell migration, enabled the inventors to identify IL4I1 as a driver of cancer cell motility. AHR-driven motility contributes to the malignant properties of gliomas and other cancer entities, but has so far mainly been linked to its upstream metabolic regulators IDOl and TDO2 (Chen et al., 2014; D'Amato et al., 2015; Gabriely et al., 2017; Novikov et al., 2016; Opitz et al., 2011; Xiang et al., 2019). The inventors show here that IL4I1 is of greater importance in glioma survival than IDOl and TDO2, and this strong negative association with glioma survival is at least in part be due to IL4I1's pro-migratory effects.

IL4I1's migratory outcomes do not only explain its importance in glioma but may also underlie its impact in metastatic cancers such as melanoma. In this context, it is important to note that unlike IDOl and TDO2, IL4I1 is secreted (Boulland et al., 2007).

Thus, IL4I1 might enhance systemic Trp-I3P catabolism, thereby contributing to a systemic pro-metastatic environment that allows metastatic cells to migrate and protects them from immune destruction. In support of this idea, cancer patients exhibit increased systemic concentrations of IL4I1-derived metabolites (Fong et al., 2011; Huang et al., 2016; Locasale et al., 2012).

In contrast to IDO1- and TDO2-derived Trp catabolites, the IL4I1-derived catabolite I3P not only activates the AHR, but also impedes the clearance of AHR ligands by inhibiting the activity of cytochrome P450 enzymes (Bittinger et al., 2003; Smirnova et al., 2016). Also H₂O₂ inhibits cytochrome P450 enzymes (Wincent et al., 2012). The dual suppression of cytochrome P450 enzymes by the two IL4I1-derived metabolites I3P and H₂O₂ may be one reason why AHR activation by IL4I1 is more pronounced than that by IDO1 or TDO2. In addition, I3P also promotes the degradation of hypoxia inducible factor 1a (HIF1a) (McGettrick et al., 2016). HIF1a competes with the AHR for dimerization with ARNT (Gabriely et al., 2017; Wang et al., 1995), and thus more ARNT is available to dimerize with the AHR when HIF1a is inhibited. The reduction in HIF1a levels may therefore additionally contribute to the I3P-mediated activation of the AHR

Until now, indole metabolites, including I3P, and their effects on AHR activity have been attributed to microbial metabolism (Aoki et al., 2018; Cervantes-Barragan et al., 2017; Dodd et al., 2017; Hubbard et al., 2015; Natividad et al., 2018; Rothhammer et al., 2018; Zelante et al., 2013). The inventors identify IL4I1 as a hitherto unknown source of I3P and its downstream metabolites IAA, I3A and ILA in humans. Thus, the inventors establish IL4I1-mediated Trp catabolism as a novel route of indole formation in eukaryotes.

The inventors also show that KynA is formed from I3P which, although reported already 30 years ago, has not received much attention since (Politi et al., 1991; Russi et al., 1989). This finding directly links IL4I1 with the generation of the AHR agonist KynA, further underpinning the importance of IL4I1 for AHR signaling. Moreover, the formation of KynA by IL4I1 has important implications for the diverse functions of this metabolite (Agudelo et al., 2018; Cervenka et al., 2017), particularly in the central nervous system (CNS) (Lemieux et al., 2015; Schwarcz et al., 2012; Stone et al., 2013). In the CNS, IL4I1's AHR-dependent outputs might mediate hitherto orphan AHR functions that cannot be assigned to IDO1 and TDO2. This concerns for instance the re-myelination of neurons: Defective myelination is the main phenotype in humans carrying an AHR loss-of-function-mutation (Mayer et al., 2019), but has so far not been linked to IDOl or TDO2. IL4I1 promotes re-myelination (Psachoulia et al., 2016), suggesting that the IL4I1-I3P-AHR axis mediates this function, and highlighting the impact of this axis beyond cancer.

Next to assigning new functions to IL4I1, its stimulatory effect on AHR also adds key aspects to IL4I1's known involvement in immunity (Molinier-Frenkel et al., 2019). Specifically, the enrichment of MDSCs and Tregs in IL4I1-expressing tumors is likely attributable to the AHR, as it promotes Treg differentiation (Esser et al., 2009; Gagliani et al., 2015; Marshall and Kerkvliet, 2010; Mezrich et al., 2010; Quintana et al., 2008) and MDSC recruitment to tumors (Neamah et al., 2019).

The co-existence of IL4I1, IDOl, and TDO2 as activators of the AHR underscores the biological importance of this metabolic checkpoint. In the context of tumor therapy, the stronger effects of IL4I1 on the AHR - as compared to IDO1 or TDO2 - highlight IL4I1 as a key drug target upstream of the AHR. It remains to be investigated whether the co-inhibition of IDOl or TDO2 (see, for example, WO 2017/107979A1) is necessary in order to avoid therapy escape in response to IL4I1 blockade (see, for example, Prendergast GC, et al.. Discovery of IDO1 Inhibitors: From Bench to Bedside. Cancer Res. 2017;77(24):6795-6811, and Cheong JE, et al. A patent review of IDOl inhibitors for cancer. Expert Opin Ther Pat. 2018 Apr;28(4):317-330). This is also critical in the context of ICB, which enhances IL4I1 levels along with IDOl and may therefore require concomitant targeting of both of these enzymes to achieve efficient de-repression of anti-tumor immunity. The inventors therefore advocate the development of IL4I1-targeting drugs as a new avenue for cancer therapy and beyond.

The present invention thus relates to the following items.
Item 1. A method for detecting and/or diagnosing cancer in a patient comprising detecting the expression or enzymatic activity of IL4I1 in a sample obtained from said patient, and diagnosing and/or detecting cancer in said patient, if said expression or enzymatic activity of said IL4I1 is increased in said patient when compared to control genes e.g, one or more housekeeping genes in said sample, or a control sample, e.g. a sample derived from a healthy subject, a group of healthy subjects, a prior sample from the same patient, a different patient or patient group.
Item 2. The method according to item 1, wherein said detection of expression and/or enzymatic activity of IL4I1 in said sample comprises the detection of the amount and/or concentration of IL4I1 and/or IL4I1 metabolites in said sample.
Item 3. The method according to claim 2, wherein said metabolites are selected from metabolites derived from the conversion of IL4I1 of phenylalanine, tyrosine and/or tryptophan, such as, for example, phenylpyruvic acid (PP), hydroxyphenylpyruvic acid (HPP), indole-3-pyruvic acid (I3P), 2-phenylacetic acid, phenyllactic acid, 4-hydroxybenzaldehyde, 2-hydroxy-2-phenylacetic acid, 4-hydroxyphenyllactic acid, and in particular the I3P derivatives indole-3-acetic acid (IAA), indole-3-aldehyde (I3A) and indole-3-lactic acid (ILA), 4-hydroxyquinoline-2-carboxylic acid (KynA), 1,3-di(1H-indole-3-yl)acetone, (3*Z*)-1-(1*H*-indole-3-yl)-3-indole-3-ylidenepropan-2-one, indole-3-carboxylic acid, oxidized indole-3-acetic acid, and indole-3-carbinol and/or the amino acids or amino acid metabolites L-valine, L-isoleucine, L-leucine, L-alanine, L-glutamic acid, L-methionine, L-glutamine, 4-methylsulfanyl-2-oxobutanoate, alpha-keto-isoleucine, alpha-ketoisovalerate, alpha-ketoisocaproic acid, L-proline, and alpha-ketoglutaric acid and any of the above in combination with ammonia and/or H₂O₂.
Item 4. The method according to any one of items 1 to 3, wherein detecting the expression and/or enzymatic activity of IL4I1 comprises the use of chromatography, NMR, metabolite sensors, antibodies, ELISAs, enzymatic assays, colorimetric assays, fluorescence assays, and/or H₂O₂ detection or ammonia detection, and/or detecting expression using genetic tools, such as chip analysis and primers and probes and PCR analysis, or detecting the amount of protein of IL4I1 using, for examples, antibodies, in samples, like biological fluids and cells/ tissue samples.
Item 5. A method for detecting increased tumor cell motility in a cancer patient comprising detecting the activity and/or expression of IL4I1 in a sample obtained from said patient, and detecting increased tumor cell motility in said patient, if said expression or enzymatic activity of said IL4I1 is increased in said patient when compared to control genes e.g, one or more housekeeping genes in said sample, or a control sample, e.g. a sample derived from a healthy subject, a group of healthy subjects, a prior sample from the same patient, a different patient or patient group.
Item 6. The method according to item 5, wherein said cancer is characterized by a modulation, such as an increase of the activity and/or expression of AHR, and/or is preferably selected from the group consisting of B cell lymphoid malignancies, such as follicular lymphoma, Hodgkin lymphoma, primary mediastinal B cell lymphoma, diffuse large B cell lymphoma, marginal zone lymphoma and chronic lymphoid leukemia, ovarian carcinomas, mesotheliomas, colon carcinomas, breast carcinomas, melanomas, glioblastomas, prostate cancer, endometrial cancer, and lung carcinomas, preferably displaying IL4I1-expressing cells, and relapsing and/or metastasizing forms thereof.
Item 7. A method for predicting or detecting the effect of cancer immunotherapy in a cancer patient comprising detecting the activity and/or expression of IL4I1 in a sample obtained from said patient, wherein if said expression or enzymatic activity of said IL4I1 is increased in said patient when compared to control genes e.g, one or more housekeeping genes in said sample, or a control sample, e.g. a sample derived from a healthy subject, a group of healthy subjects, a prior sample from the same patient, a different patient or patient group is predictive for and/or indicates a reduced effect and/or immune evasion of said cancer immunotherapy in said patient.
Item 8. The method according to item 7, wherein said increase of said activity and/or expression of said IL4I1 is detected in response to an immunotherapy in said patient.
Item 9. A method for detecting resistance against a cancer treatment comprising immunotherapy, such as for example immune checkpoint blockade (ICB), in a patient comprising detecting the activity and/or expression of IL4I1 in a sample obtained from said patient, and detecting resistance against a cancer treatment comprising ICB in said patient, if said expression or enzymatic activity of said IL4I1 is increased in said patient when compared to control genes e.g, one or more housekeeping genes in said sample, or a control sample, e.g. a sample derived from a healthy subject, a group of healthy subjects, a prior sample from the same patient, a different patient or patient group.
Item 10. The method according to item 9, wherein said ICB treatment comprises the use of IDOl inhibitors and/or antibodies selected from anti-CTLA-4, anti-PD-1, anti-PDL-1, anti LAG3 and anti BTLA.
Item 11. A method for predicting survival in a cancer patient comprising detecting the activity and/or expression of IL4I1 in a sample obtained from said patient, wherein said expression or enzymatic activity of said IL4I1 is increased in said patient when compared to control genes e.g, one or more housekeeping genes in said sample, or a control sample, e.g. a sample derived from a healthy subject, a group of healthy subjects, a prior sample from the same patient, a different patient or patient groupis predictive for a reduced survival in said patient.
Item 12. The method according to any one of items 1 to 11, wherein said detecting of said activity comprises detecting IL4I1 metabolites, in particular IL4I1 tryptophan metabolites such as, for example I3P or I3P derived metabolites.
Item 13. The method according to any one of items 1 to 12, wherein detecting the enzymatic activity of IL4I1 comprises the use of chromatography, NMR, metabolite sensors, antibodies, ELISAs, enzymatic assays, colorimetric assays, fluorescence assays, and/or H₂O₂ or ammonia detection, and/or detecting expression using genetic tools, such as chip analysis and primers and probes and PCR analysis, or detecting the amount of protein of IL4I1 using, for examples, antibodies, in samples, like biological fluids and cells/ tissue samples.
Item 14. The method according to any one of items 1 to 13, wherein said detection of said activity comprises detecting the activity and/or expression of AHR, wherein an increase in said activity and/or expression of AHR when compared to a control is indicative for an increase of the activity of IL4I1.
Item 15. The method according to any one of items 1 to 14, wherein said biological sample is selected from a suitable sample comprising biological fluids, mammalian, for example human, cells, tissues, whole blood, cell lines, cellular supernatants, primary cells, IPSCs, hybridomas, recombinant cells, stem cells, and cancer cells, bone cells, cartilage cells, nerve cells, glial cells, epithelial cells, skin cells, scalp cells, lung cells, mucosal cells, muscle cells, skeletal muscles cells, striated muscle cells, smooth muscle cells, heart cells, secretory cells, adipose cells, blood cells, erythrocytes, basophils, eosinophils, monocytes, lymphocytes, T-cells, B-cells, neutrophils, NK cells, regulatory T-cells, dendritic cells, Th17 cells, Th1 cells, Th2 cells, myeloid cells, macrophages, monocyte derived stromal cells, bone marrow cells, spleen cells, thymus cells, pancreatic cells, oocytes, sperm, kidney cells, fibroblasts, intestinal cells, cells of the female or male reproductive tracts, prostate cells, bladder cells, eye cells, corneal cells, retinal cells, sensory cells, keratinocytes, hepatic cells, brain cells, kidney cells, and colon cells, and the transformed counterparts of said cells or tissues, and in particular cancer cells that do not derive from the immune system.
Item 16. The method according to any one of items 1 to 15, wherein said control sample comprises control genes e.g, one or more housekeeping genes in said sample, or is a control sample derived for example from a healthy subject or group of healthy subjects, a prior sample from the same patient, a different patient or patient group.
Item 17. The method according to any one of items 1 to 16, wherein said cancer is characterized by a modulation, such as an increase of the activity and/or expression of AHR, and/or is preferably selected from the group consisting of B cell lymphoid malignancies, such as follicular lymphoma, Hodgkin lymphoma, primary mediastinal B cell lymphoma, diffuse large B cell lymphoma, marginal zone lymphoma and chronic lymphoid leukemia, ovarian carcinomas, mesotheliomas, colon carcinomas, breast carcinomas, melanomas, glioblastomas, prostate cancer, endometrial cancer, and lung carcinomas, preferably displaying IL4I1-expressing cells, and relapsing and/or metastasizing forms thereof.
Item 18. The method according to any one of items 1 to 17, further comprising the step of a stratification of said patient to a disease and/or group.
Item 19. A diagnostic kit comprising materials for performing a method according to any one of items 1 to 18 in one or separate containers, optionally together with auxiliary agents and/or instructions for performing said method.
Item 20. Use of a diagnostic kit according to item 19 for a method according to any one of items 1 to 19.
Item 21. A method for treating and/or preventing an IL4I1-related disease or condition in a cell, for example in a patient in need of said treatment, comprising performing a method according to any one of items 1 to 18, and providing a suitable treatment to said patient, wherein said treatment is based, at least in part, on the results of the method according to the present invention.

**Table 1 - Amino acids and metabolites converted and modulated by IL4I1**

| **Chemical Name** | **InChI Key** | **PubChem CID** |
|---|---|---|
| 3-Phenylpyruvic acid (PP) | BTNMPGBKDVTSJY- UHFFFAOYSA-N | 997 |
| | InChI=1 S/C9H8O3/c10-8(9(11)12)6-7-4-2-1-3-5-7/h1-5H,6H2,(H,11,12) | |
| Indole-3-pyruvic acid (I3P) | RSTKLPZEZYGQPY- UHFFFAOYSA-N | 803 |
| | InChI=1S/C11H9NO3/c13-10(11(14)15)5-7-6-12-9-4-2-1-3-8(7)9/h1-4,6,12H,5H2,(H,14,15) | |
| 4-Hydroxyphenylpyruvic acid (HPP) | KKADPXVIOXHVKN- UHFFFAOYSA-N | 979 |
| | InChI=1 S/C9H8O4/c10-7-3-1-6(2-4-7)5-8(11)9(12)13/h1-4,10H,5H2,(H,12,13) | |
| 2-Phenylacetic acid | WLJVXDMOQOGPHL- UHFFFAOYSA-N | 999 |
| | InChI=1 S/C8H8O2/c9-8(10)6-7-4-2-1-3-5-7/h1-5H,6H2,(H,9,10) | |
| 4-Hydroxybenzaldehyde | RGHHSNMVTDWUBI- UHFFFAOYSA-N | 126 |
| | InChI=1 S/C7H6O2/c8-5-6-1-3-7(9)4-2-6/h1-5,9H | |
| 2-Hydroxy-2-phenylacetic acid | IWYDHOAUDWTVEP- UHFFFAOYSA-N | 1292 |
| | InChI=1 S/C8H8O3/c9-7(8(10)11)6-4-2-1-3-5-6/h1-5,7,9H,(H,10,11) | |
| 3-Indoleacetic acid (IAA) | SEOVTRFCIGRIMH- UHFFFAOYSA-N | 802 |
| | InChI=1 S/C 1 0H9NO2/c12-10(13)5-7-6-11-9-4-2-1-3-8(7)9/h1-4,6,11H,5H2,(H,12,13) | |
| 1H-Indole-3-aldehyde (I3A) | OLNJUISKUQQNIM- UHFFFAOYSA-N | 10256 |
| | InChI=1 S/C9H7NO/C11-6-7-5-10-9-4-2-1-3-8(7)9/h1-6,10H | |
| Indole-3-lactic acid (ILA) | XGILAAMKEQUXLS- UHFFFAOYSA-N | 92904 |
| | InChI=1S/C11H11NO3/c13-10(11(14)15)5-7-6-12-9-4-2-1-3-8(7)9/h1-4,6,10,12-13H,5H2,(H,14,15) | |
| 4-Hydroxyquinoline-2-carboxylic acid, kynurenic acid (KynA) | HCZHHEIFKROPDY- UHFFFAOYSA-N | 3845 |
| | InChI=1S/C10H7NO3/c12-9-5-8(10(13)14)11-7-4-2-1-3-6(7)9/h1-5H,(H,11,12)(H,13,14) | |
| 1,3-di(1H-indol-3-yl)acetone | OULRFLUQRMGBEN- UHFFFAOYSA-N | 11483104 |
| | InChI=1 S/C19H16N2O/c22-15(9-13-11-20-18-7-3-1-5-16(13)18)10-14-12-21-19-8-4-2-6-17(14)19/h1-8,11-12,20-21H,9-10H2 | |
| (3*Z*)-1-(1*H*-indol-3-yl)-3-indol-3-ylidenepropan-2-one | BHYVQPWGWDXGBR- UKTHLTGXSA-N | 57345798 |
| | InChI=1S/C19H14N2O/c22-15(9-13-11-20-18-7-3-1-5-16(13)18)10-14-12-21-19-8-4-2-6-17(14)19/h1-9,11-12,21H,10H2/b13-9+ | |
| L-valine | KZSNJWFQEVHDMF- BYPYZUCNSA-N | 6287 |
| | InChI=1S/C5H11NO2/c1-3(2)4(6)5(7)8/h3-4H,6H2,1-2H3,(H,7,8)/t4-/m0/s1 | |
| L-isoleucine | AGPKZVBTJJNPAG- | 6306 |
| | WHFBIAKZSA-N | |
| | InChI=1 S/C6H13NO2/c1-3-4(2)5(7)6(8)9/h4-5H,3,7H2,1-2H3,(H,8,9)/t4-,5-/m0/s1 | |
| L-leucine | ROHFNLRQFUQHCH- YFKPBYRVSA-N | 6106 |
| | InChI=1 S/C6H13NO2/c1-4(2)3-5(7)6(8)9/h4-5H,3,7H2,1-2H3,(H,8,9)/t5-/m0/s1 | |
| L-alanine | QNAYBMKLOCPYGJ- REOHCLBHSA-N | 5950 |
| | InChI=1 S/C3H7NO2/c1-2(4)3 (5)6/h2H,4H2,1H3,(H,5,6)/t 2-/m0/s1 | |
| L-glutamic acid | WHUUTDBJXJRKMK- VKHMYHEASA-N | 33032 |
| | InChI=1S/C5H9NO4/c6-3(5(9)10)1-2-4(7)8/h3H,1-2,6H2,(H,7,8)(H,9,10)/t3-/m0/s1 | |
| L-methionine | FFEARJCKVFRZRR- BYPYZUCNSA-N | 6137 |
| | InChI=1S/C5H11NO2S/c1-9-3-2-4(6)5(7)8/h4H,2-3,6H2,1H3,(H,7,8)/t4-/m0/s1 | |
| L-glutamine | ZDXPYRJPNDTMRX- VKHMYHEASA-N | 5961 |
| | InChI=1 S/C5H10N2O3/c6-3(5(9)10)1-2-4(7)8/h3H,1-2,6H2,(H2,7,8)(H,9,10)/t3-/m0/s1 | |
| 4-methylsulfanyl-2-oxobutanoate | SXFSQZDSUWACKX- UHFFFAOYSA-M | 4584184 |
| | InChI=1 S/C5H8O3S/c1-9-3-2-4(6)5(7)8/h2-3H2,1H3,(H,7,8)/p-1 | |
| Alpha-Keto-isoleucine | JVQYSWDUAOAHFM- BYPYZUCNSA-N | 439286 |
| | InChI=1S/C6H10O3/c1-3-4(2)5(7)6(8)9/h4H,3H2,1-2H3,(H,8,9)/t4-/m0/s1 | |
| alpha- Ketoisovalerate | QHKABHOOEWYVLI- UHFFFAOYSA-N | 49 |
| | InChI=1S/C5H8O3/c1-3(2)4(6)5(7)8/h3H,1-2H3,(H,7,8) | |
| alpha-Ketoisocaproic acid | BKAJNAXTPSGJCU- UHFFFAOYSA-N | 70 |
| | InChI=1S/C6H1003/c1-4(2)3-5(7)6(8)9/h4H,3H2,1-2H3,(H,8,9) | |
| L-proline | ONIBWKKTOPOVIA- BYPYZUCNSA-N | 145742 |
| | InChI=1 S/C5H9NO2/c7-5(8)4-2-1-3-6-4/h4,6H, 1-3H2,(H,7,8)/t4-/m0/s1 | |
| alpha-ketoglutaric acid | KPGXRSRHYNQIFN- UHFFFAOYSA-N | 51 |
| | InChI=1 S/C5H6O5/c6-3(5(9)10)1-2-4(7)8/h1-2H2,(H,7,8)(H,9,10) | |
| (2Z)-3H-indol-3-ylidene-ethanoic acid (oxidized indole-3-acetic acid) | ZNWKELRZJMEGNL- FNORWQNLBJ | n.a. |
| | InChI=1/C10H7NO2/c12-10(13)5-7-6-11-9-4-2-1-3-8(7)9/h1-6H,(H,12,13) | |
| Indole-3-carboxylic acid | KMAKOBLIOCQGJP- UHFFFAOYAT | 69867 |
| | InChI=1/C9H7NO2/c11-9(12)7-5-10-8-4-2-1-3-6(7)8/h1-5,10H,(H,11,12) | |
| 4-Hydroxyphenyllactic acid | JVGVDSSUAVXRDY- UHFFFAOYAV | 9378 |
| | InChI=1/C9H10O4/c10-7-3-1-6(2-4-7)5-8(11)9(12)13/h1-4,8,10-11H,5H2,(H,12,13) | |
| Indole-3-carbinol | IVYPNXXAYMYVSP- UHFFFAOYAO | 3712 |
| | InChI=1/C9H9NO/c11-6-7-5-10-9-4-2-1-3-8(7)9/h1-5,10-11H,6H2 | |
| Phenyllactic acid | VOXXWSYKYCBWHO- UHFFFAOYAI | 3848 |
| | InChI=1/C9H10O3/c10-8(9(11)12)6-7-4-2-1-3-5-7/h1-5,8,10H,6H2,(H,11,12) | |

**Table 2 - Age adjusted multivariate cox proportional hazards of high expression of IL4I1, IDOl or TDO2 in GBM and LGG TCGA cohorts**

| Tumor | Enzyme | coef | Cox proportional hazard | se.coef. | z | P-value |
|---|---|---|---|---|---|---|
| GBM | IL4I1 | 0.8247 | 2.281 | 0.206 | 3.999 | 6.391E-05 |
| GBM | IDOl | 0.1501 | 1.162 | 0.184 | 0.816 | 0.414 |
| GBM | TDO2 | 0.4113 | 1.509 | 0.202 | 2.040 | 0.041 |
| LGG | IL4I1 | 1.2068 | 3.343 | 0.201 | 6.017 | 1.777E-09 |
| LGG | IDOl | 0.8895 | 2.434 | 0.193 | 4.600 | 4.219E-06 |
| LGG | TDO2 | 0.4982 | 1.646 | 0.250 | 1.991 | 0.046 |

The invention shall now be further described in the following examples with reference to the accompanying figures, nevertheless, without being limited thereto. For the purposes of the present invention, all references as cited herein are incorporated by reference in their entireties.

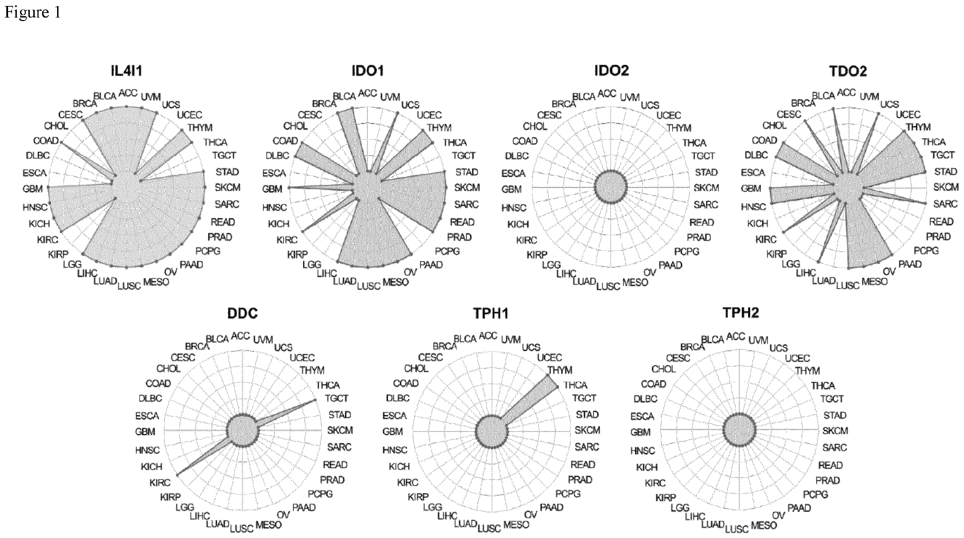

Figure 1 shows that a pan-tissue AHR signature reveals a strong association of *IL4I1* with AHR activity. Spider plots show the incidence of the seven Trp-degrading enzymes in AHR-associated modules (AAMs), depicted by the shaded area. The dots on the axes have values of either 0 (inner circle - absent) or 1 (outer circle - present). The shaded area is formed by connecting the dots representative for each tumor type.

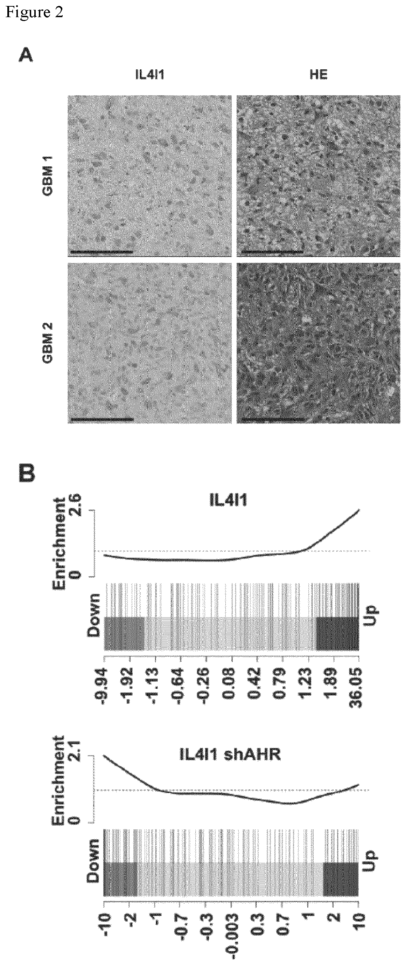

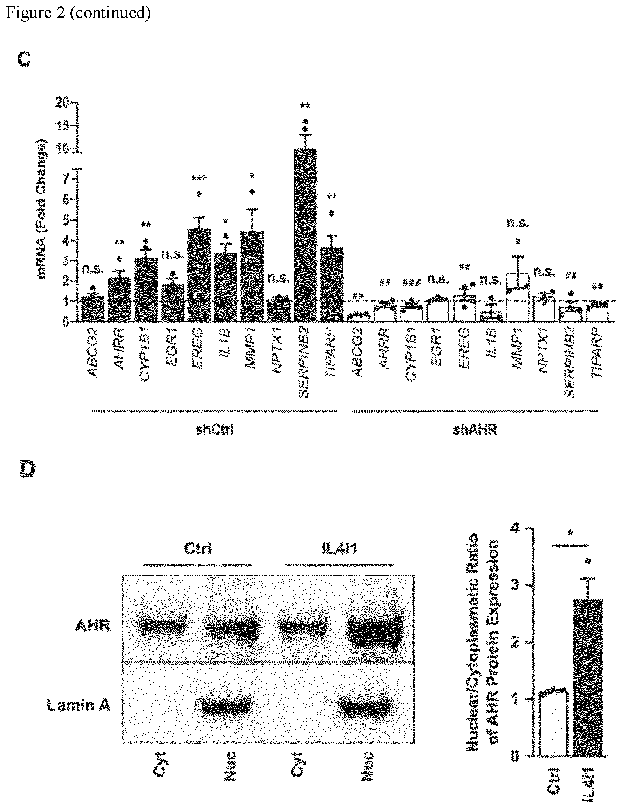

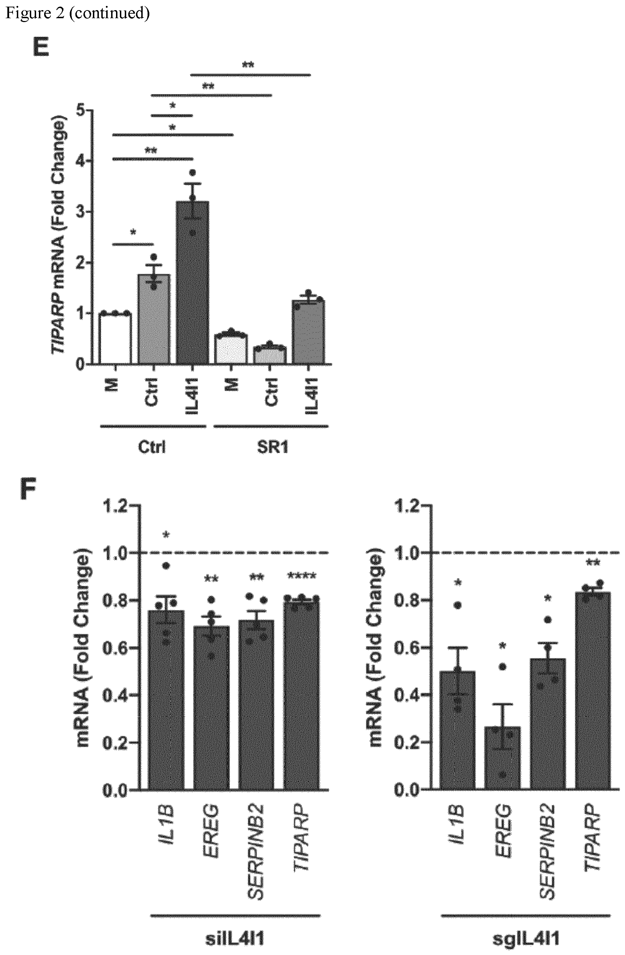

Figure 2 shows that IL4I1 activates the AHR. (A) IL4I1 expression in two human glioblastoma (GBM) specimens. IL4I1 staining (left), HE staining (right). Magnification 20x, scale bar 100 µm. (B) Barcode plots showing the regulation of the AHR signature in shCtrl (top) and shAHR (bottom) U-87MG glioblastoma cells expressing ectopic IL4I1, cultured for 120 h *(n* = 3). (C) mRNA expression of selected AHR target genes in shCtrl and shAHR U-87MG cells expressing ectopic IL4I1, relative to shCtrl U-87MG cells without IL4I1 expression (dashed line), cultured for 120 h *(n* = 3 for *EGR1, IL1B, MMP1; n* = 4 for all other genes). (D) Immunoblot (left) and quantification (right) of nuclear to cytoplasmic ratio of AHR protein expression in LN-229 cells upon 4 h treatment with supernatants of Ctrl and IL4I1-expressing U-251MG cells, cultured for 120 h *(n* = 3). (E) *TIPARP* mRNA expression in U-87MG cells treated for 24 h with 1 µM SR1 and medium (M) or supernatants of Ctrl and IL4I1-expressing U-87MG cells, relative to U-87MG cells treated with medium containing DMSO *(n* = 3). (F) mRNA expression of selected AHR target genes in CAS-1 cells. Left plot: RNA interference of *IL4I1* (siIL4I1), relative to cells treated with siCtrl (dashed line), cultured for 72 h (*n* = 3). Right plot: downregulation of *IL4I1* using CRISPR/Cas9 (sgIL4I1), relative to cells treated with non-targeting control sgRNA (dashed line), cultured for 72 h (*n* = 4). *n* represents the number of independent experiments. Data are presented as mean ± S.E.M and were analyzed by two-tailed paired student's *t*-test (C-F). * *P* < 0.05, ** *P* < 0.01, *** *P* < 0.001.

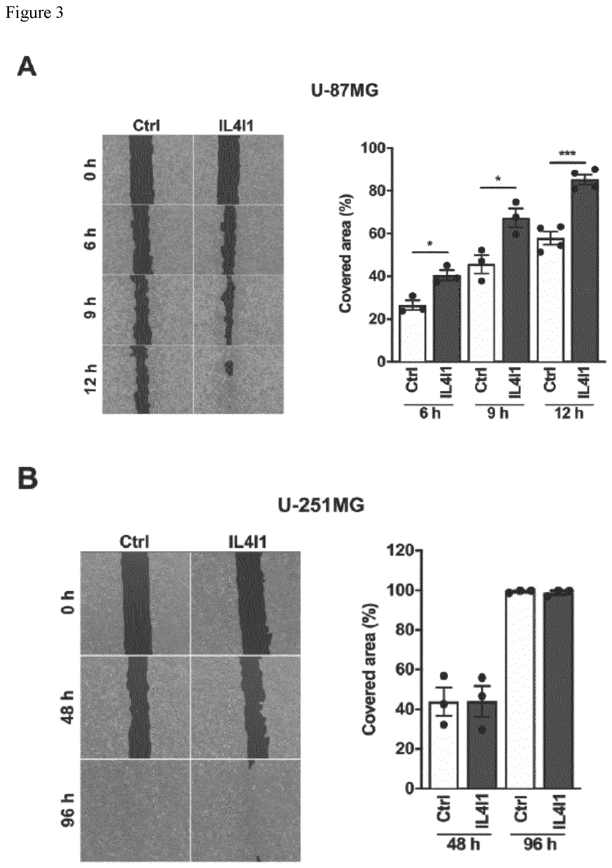

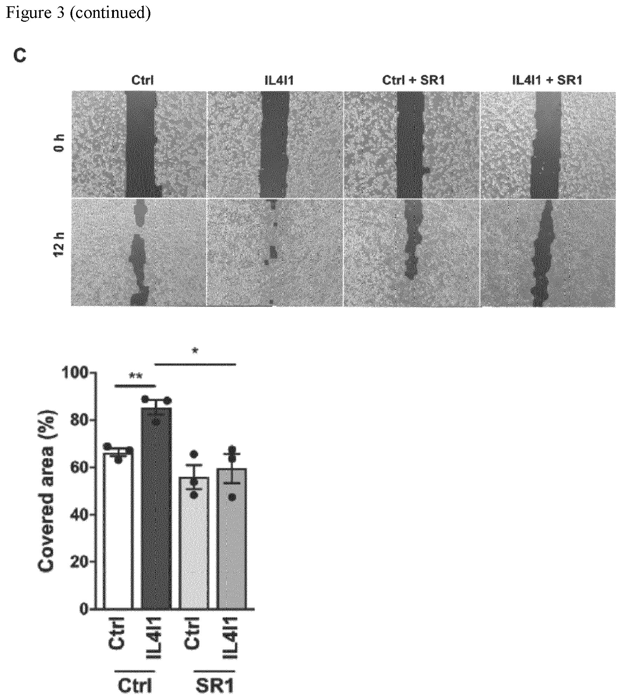

Figure 3 shows that IL4I1 promotes AHR-mediated pro-tumor effects. (A, B) Representative images (left) and quantification (right) of the percentage of the area covered by Ctrl and IL4I1-expressing U-87MG (A) and U-251MG (B) cells in a wound healing assay at the indicated time points (*n* = 3 for 6 h, 9 h and 12 h in U-87MG and 48 h and 96 h in U-251MG, *n* = 4 for 12 h in U-87MG). (C) Representative images (left) and quantification (right) of the percentages of the area covered by Ctrl and ectopically IL4I1-expressing U-87MG cells in the absence or presence of AHR inhibition with 1 µM SR1, in a wound healing assay at the indicated time points (*n* = 3). (D) Kaplan Meier curves of the overall survival outcome of TCGA GBM patients when divided into groups of high and low expression of *IL4I1, IDO1* and *TDO2.* The p-values represent the probability of the age adjusted cox proportional hazard when comparing the group of high expression to that of low expression of the enzymes. (E) Overall survival probability as described in D but for the TCGA LGG patient cohort Data are presented as mean ± S.E.M and were analyzed by two-tailed unpaired student's *t*-test (A, B, C). * *P* < 0.05, ** *P* < 0.01, **** *P* < 0.0001.

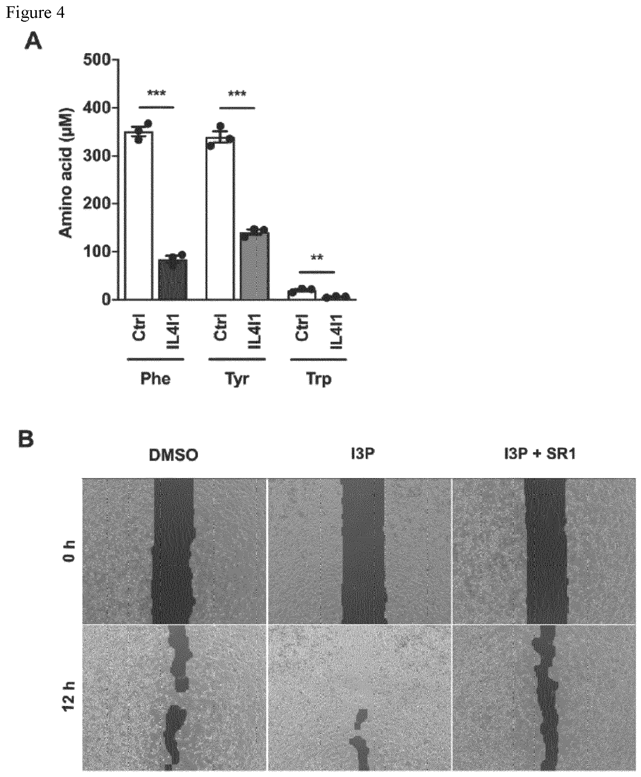

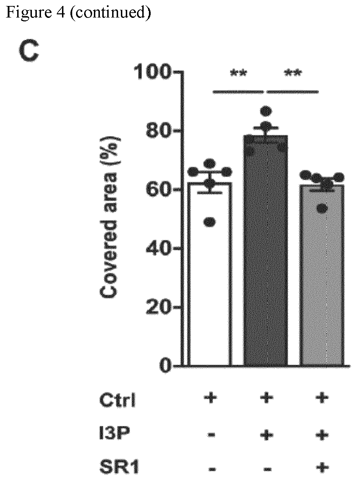

Figure 4 shows that indole-3-pyruvate is the key metabolite mediating IL4I1-driven AHR activity and motility. (A) Concentration of Phe, Tyr and Trp in supernatants of Ctrl and IL4I1-expressing U-87MG cells, cultured for 120 h (*n* = 3). (B-C) Representative images (B) and quantification (C) of the percentage of the area covered by U-87MG cells treated with 25 µM I3P and 1 µM SR1, in a wound healing assay at the indicated time points (*n* = 5). *n* values represent the number of independent experiments. Data are presented as mean ± S.E.M and were analyzed by two-tailed unpaired student's *t*-test (C). * *P* < 0.05, ** *P* < 0.01, *** *P* < 0.001.

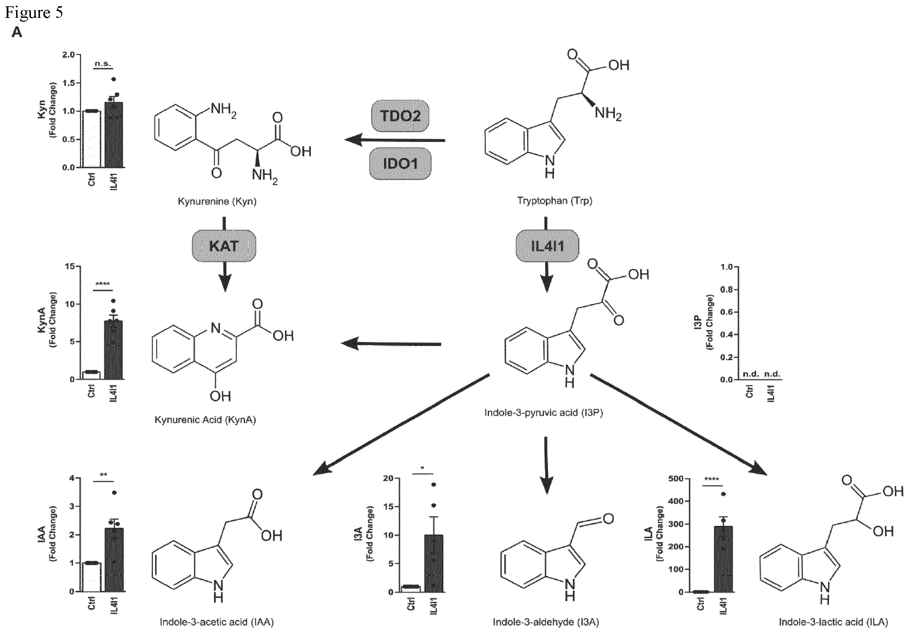

Figure 5 shows that indole-3-pyruvate gives rise to kynurenic acid and indole-derivatives. IL4I1 activity generates indole-metabolites and KynA. Relative abundance of Kyn, KynA, I3P, IAA, I3A and ILA in supernatants of Ctrl and IL4I1-expressing U-87MG cells (120 h) (*n* = 6 for Kyn, KynA, I3P and IAA; *n* = 5 for I3A and ILA). *n* values represent the numbers of independent experiments. Data are presented as mean ± S.E.M and were analyzed by one sample t-test * *P* < 0.05, ** *P* < 0.01, *** *P* < 0.001, **** *P* < 0.0001. n.s., not significant; n.d., not detected.

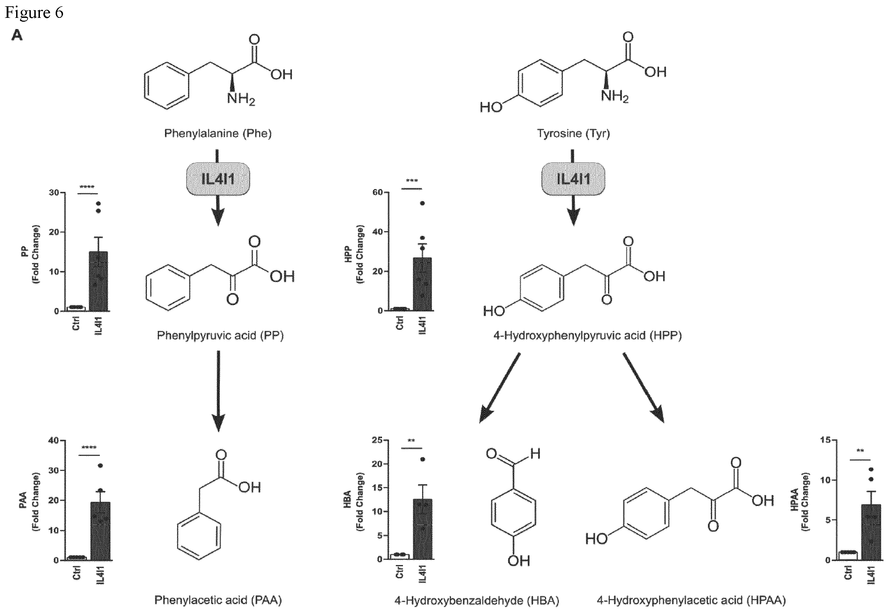

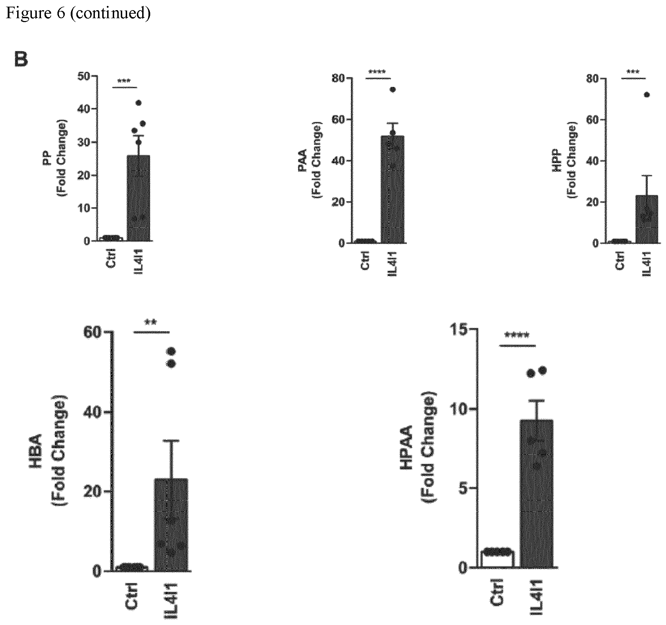

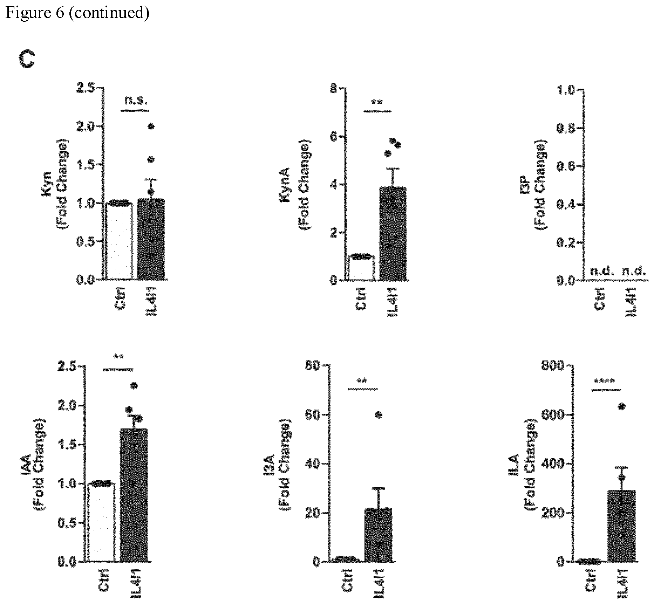

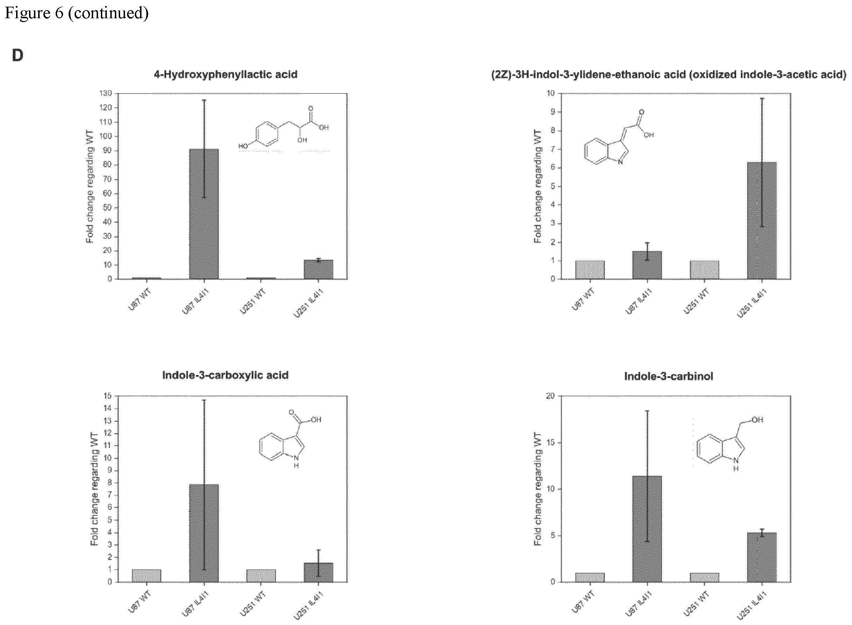

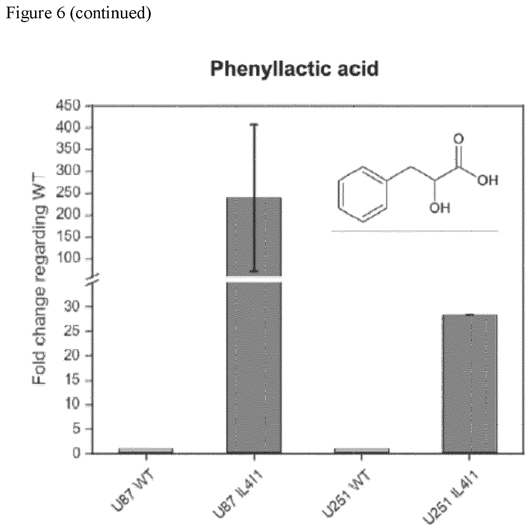

Figure 6 shows that IL4I1 activity generates metabolites derived from aromatic amino acids . (A) Relative abundance of PP, PAA, HPP, HBA and HPAA in supernatants of Ctrl and IL4I1-expressing U-87MG cells (120 h) *(n* = 6 for PP and HPP; *n* = 5 for PAA and HPAA; *n* = 4 for HBA). (B) Relative abundance of PP, PAA, HPP, HBA and HPAA in supernatants of Ctrl and IL4I1-expressing U-251MG cells (120 h) (*n* = 6 for PP, HPP, HBA; *n* = 5 for PAA and HPAA). (C) Relative abundance of Kyn, KynA, I3P, IAA, I3A and ILA in supernatants of Ctrl and IL4I1-expressing U-251MG cells (120 h) (*n* = 6 for Kyn, KynA, I3P, IAA, I3A; *n* = 5 for ILA). (D) Relative abundance of 4-hydroxyphenyllactic acid, indole-3-carboxylic acid, oxidized indole-3-acetic acid, phenyllactic acid and indole-3-carbinol in concentrated supernatants of Ctrl and IL4I1-expressing U87-MG and U-251MG cells (120 h) (*n* = 6) n values represent the numbers of independent experiments. Data are presented as mean ± S.E.M and were analyzed by two-tailed paired student's t-test. * *P* < 0.05, ** *P* < 0.01, *** *P* < 0.001, **** *P <* 0.0001. n.s., not significant; n.d., not detected.

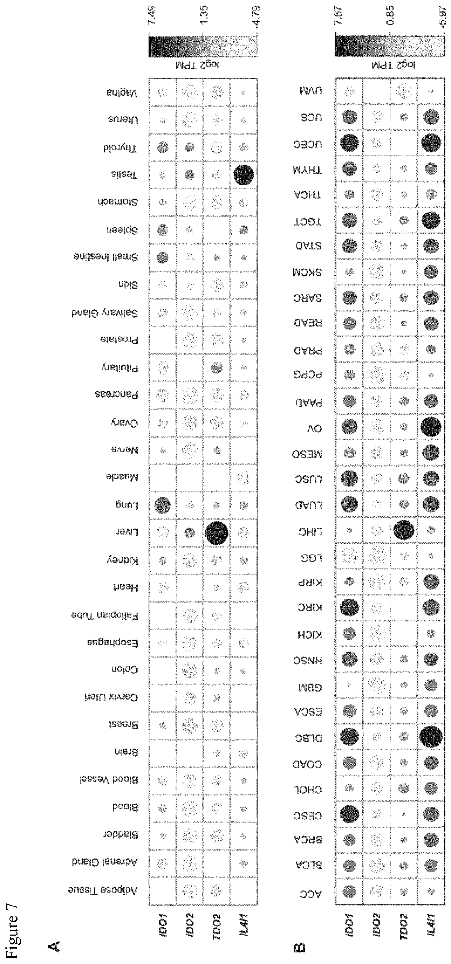

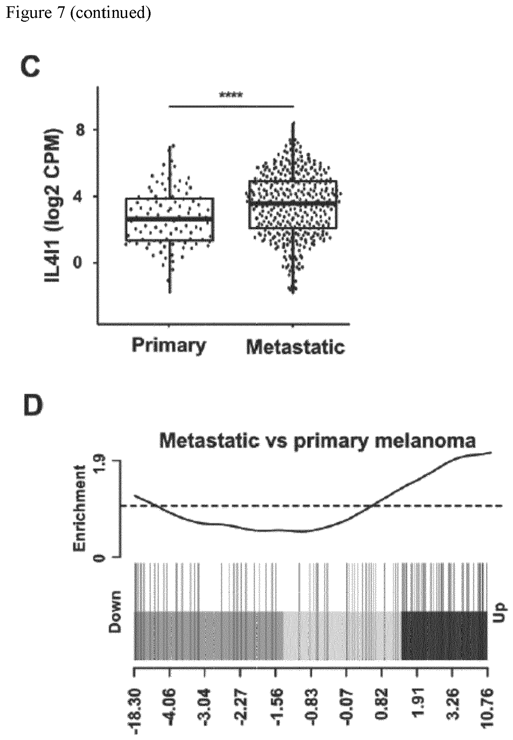

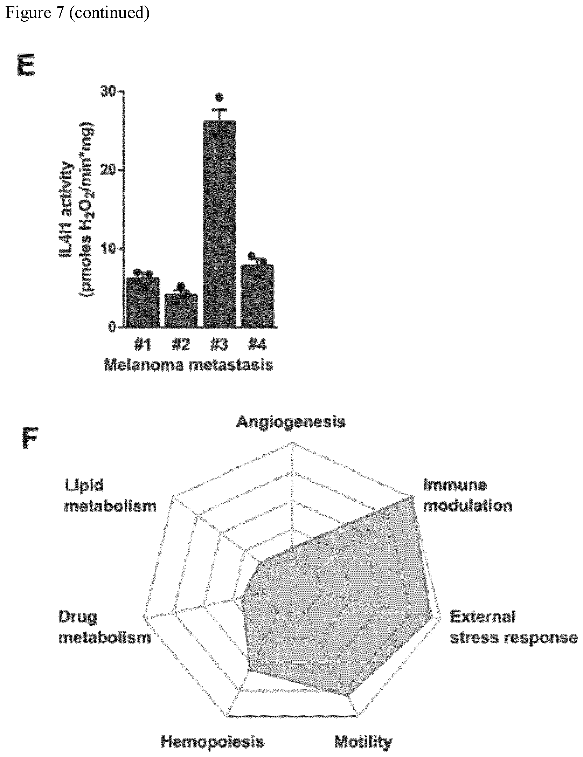

Figure 7 shows that *IL4I1* expression is increased in cancer and metastasis. (A) Heatmap of the median log2 TPM of *IDO1, IDO2, TDO2* and *IL4I1* expression in the Genotype-Tissue Expression (GTEx) dataset comprising 30 non-diseased tissues. Empty cells denote that no expression was detected. Dot size and colors correspond to the expression level, blue denoting low expression and red denoting high expression levels. (B) Heatmap of the median log2 TPM of the enzymes as in A of 32 TCGA tumors. (C) *IL4I1* expression as log2 counts per million (log2 CPM) comparing primary with metastatic melanoma of the TCGA cohort (unpaired Wilcoxon rank test; **** = *P* < 0.0001). (D) Barcode plot showing AHR activation in metastatic versus primary melanoma patients of the TCGA cohort. (E) IL4I1 activity in metastatic melanoma resected from four individual patients in the presence of 10 mM Phe. Data are mean ± SD (*n* = 3, technical replicates per patient). (F) Spider plot showing the incidence of gene ontologies enriched in AAMs containing IL4I1.

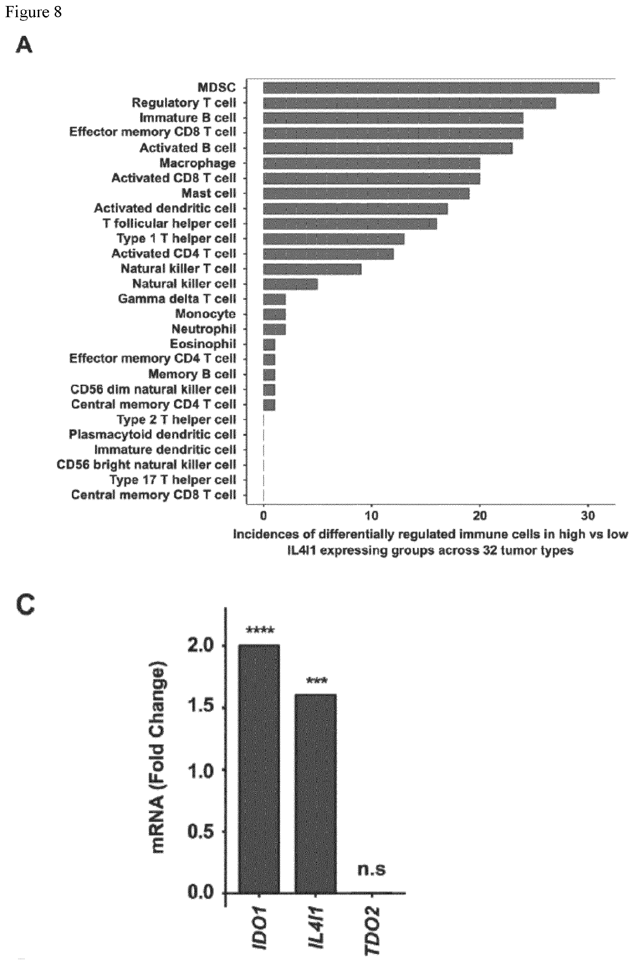

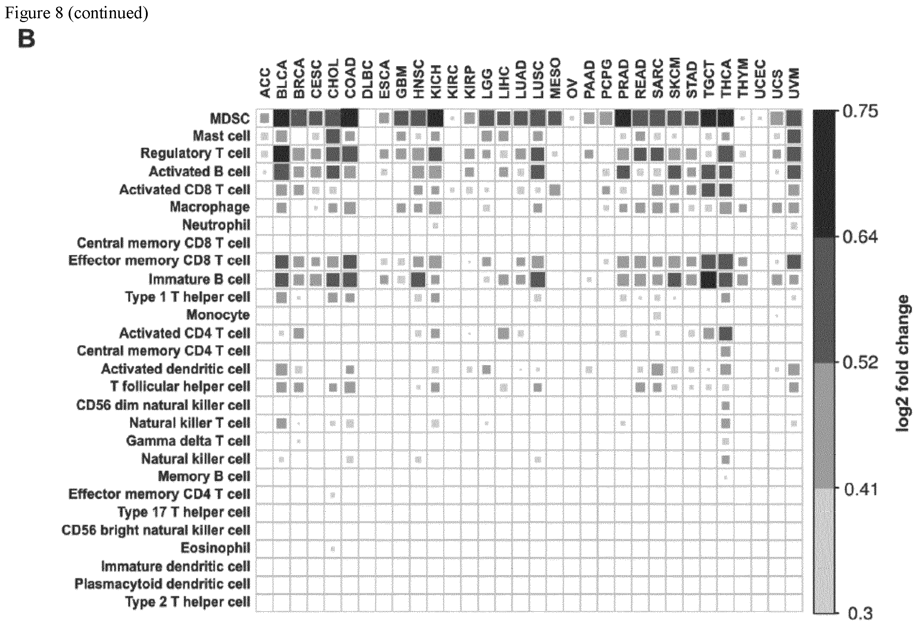

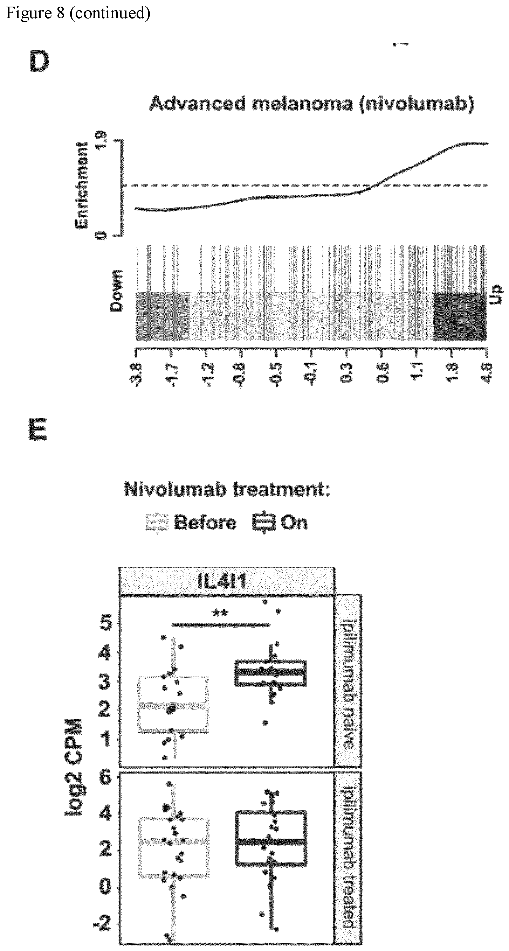

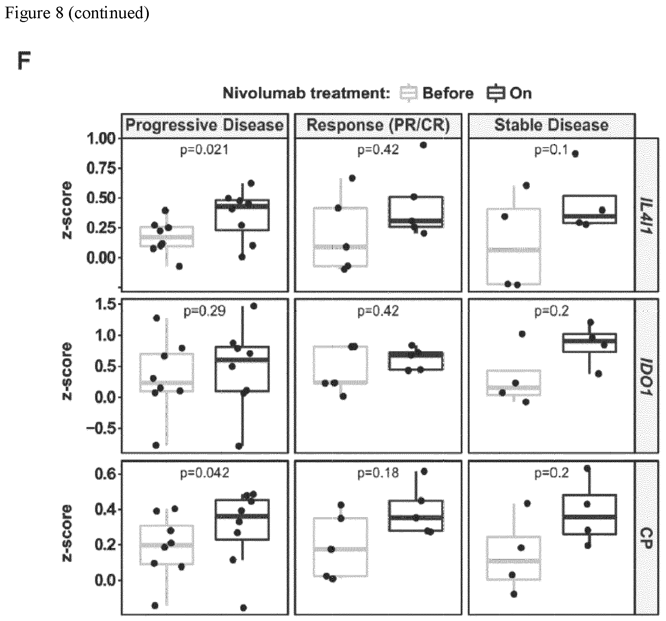

Figure 8 shows that IL4I1 is a metabolic immune checkpoint. (A) Bar graph representation of the significant incidences of differentially regulated immune cells infiltrating high vs low IL4I1 expressing TCGA tumors. (B) Heatmap representation showing the significant log2 fold change of immune cell infiltration in TCGA tumors with high vs low *IL4I1* expression separated by median expression of *IL4I1.* (C) Fold change of *IDOl, IL4I1* and *TDO2* expression in advanced melanoma patients before and after receiving nivolumab (anti-PD-1 mAb) treatment (GSE91061). (D) Barcode plot of AHR activation after nivolumab treatment in advanced melanoma patients. (E) *IL4I1* expression levels as log2 CPM in advanced melanoma in patients either naive to ipilimumab (top) or having received ipilimumab preceding nivolumab treatment (bottom); before nivolumab therapy (light gray) and after receiving nivolumab (dark gray). The p-value was estimated using a paired Wilcoxon sum ranked test. (F) *IL4I1, IDOl* and checkpoint (CP) expression reported as z-scores in advanced melanoma in patients naive to ipilimumab before (light gray) and after (dark gray) receiving nivolumab treatment, grouped according to treatment response as progressive disease (left), partial or complete response (PR/CR, middle) and stable disease (right Unless otherwise stated, n values represent independent experiments. Data are presented as mean ± S.E.M and were analyzed by one-way ANOVA with Dunnett's multiple comparisons test. *** *P* < 0.001, **** *P* < 0.0001. n.s., not significant.

### Examples

### Material and Methods

### Microarray and RNA-seq data analysis

Array datasets - For the microarray datasets generated from the different experimental conditions in this study, the Affymetrix WT PLUS Reagent Kit was used to generate labeled ss-cDNA from input amounts of 100 ng total RNA. 5.5 µg of fragmented and labeled ss-cDNA were hybridized for 17 h at 45 °C on Affymetrix Human Gene 2.0 ST Arrays according to the manufacturer's instructions. Gene Expression Microarrays were scanned using the Affymetrix GeneChip® Scanner 3000 according to the GeneChip® Expression Wash, Stain and Scan Manual for Cartridge Arrays (P/N 702731). The Affymetrix microarray chips "human gene 2.0 ST" were annotated using NetAffx and analyzed using the *oligo* package (Carvalho et al., 2007). Other Affymetrix chips were analyzed using the *affy* and *affycoretools* packages (Gautier et al., 2004). Raw CEL files were imported from disk or downloaded from GEO using GEOquery (Davis and Meltzer, 2007), followed by RMA normalization and summary.

RNA-seq datasets - Raw counts and metadata were downloaded from GEO using GEOquery (Davis and Meltzer, 2007). The harmonized HT-Seq counts and Fragments Per Kilobase of transcript per Million mapped reads (FPKM) of TCGA datasets were downloaded using TCGAbiolinks (Colaprico et al., 2016) from Genomic Data Commons (GDC) (https://gdc.cancer.gov/), and only patients with the identifier "primary tumor" were retained, with the exception of melanoma that was split into datasets for primary and metastatic cohorts. The FPKM values were converted to transcripts per million (TPMs) (Li and Dewey, 2011). TPM data of normal tissues were downloaded from the Genotype-Tissue Expression (GTEx) dataset (https://gtexportal.org/home/). All TPM values were log2 transformed. Raw counts of the Riaz et al. (2007) (Riaz et al., 2017) advanced melanoma dataset were downloaded from https://github.com/riazn/bms038_analysis, and based on principle component analysis one outlier patient pair was removed (Pt-84). RNA-seq counts were saved as DGELists (Robinson et al., 2010). Genes with less than 10 counts were filtered followed by trimmed mean of M values (TMM) normalization (Robinson and Oshlack, 2010) and variance modeling using *voom* (Law et al., 2014).

### Survival analysis

Patients of the TCGA cohorts were grouped into high or low gene expression groups by estimating a Monte-Carlo based maximal selected ranked statistics (Hothorn and Zeileis, 2008). Survival difference between the groups was estimated by fitting an age adjusted cox proportional hazard model. Kaplan-Meier curves were used for visualizing the fitted cox proportional hazard models. The reported p-value is that of the proportional hazard ratio of the high versus low group comparison.

### Defining infiltrating immune populations and estimating the immunophenogram scores

Metagenes describing 28 different immune populations were retrieved from Charoentong et al. (Charoentong et al., 2017). Sample-wise GSVA scores were generated for each tumor type as previously described. The difference between the infiltrating cells in the tumors separated by median *IL4I1* expression (high vs low) was performed using the *limma* package (Ritchie et al., 2015). Only populations showing a log2 fold change of at least 0.3 and an adjusted p-value of 0.05 were considered significant. The scores of the four compartments of the immunophenogram (antigen processing machinery (MHC), checkpoints (CP), effector cells (EC) and suppressor cells (SC)) were generated using the pipeline described by the authors of Charoentong et al. (Charoentong et al., 2017). The CP values generated are a negative weighted z-score, which corresponds to high expression of suppressive molecules and low expression of effector molecules. To allow easier comparison of CP values in the response groups before and after nivolumab treatment, the negative weighted z-scores were multiplied by -1. After this multiplication step, positive CP values will indicate high expression of suppressive molecules and low expression of effector molecules, which can be easily compared to expression levels of *IL4I1* and *IDOl* in the same groups. The CP molecules and their effect on immune response are reported in Table S8.

### Gene correlation networks associated with AHR activity

The normalized DGELists of TCGA tumors were used for weighted gene co-expression network analysis (WGCNA) (Langfelder and Horvath, 2008). Soft thresholds were estimated for signed hybrid networks in single block settings. WGCNA was run using bi-weighted correlations and Eigen genes representing the first principle components of each module were returned. Selection of WGCNA modules associated with AHR activity was conducted by performing a global test (Goeman and Finos, 2012; Goeman et al., 2004) using the AHR activity as the response and the WGCNA modules as the model predictors. AHR activity was corelated with WGCNA modules using Pearson correlation. Modules that overlapped the global test and the Pearson correlation results, with a p-value of 0.05 or less in both tests, were selected as the AHR-associated modules (AAMs). Modules with both positive and negative associations with AHR activity were considered.

### Cell culture

U-87MG were obtained from ATCC. CAS-1 and U-251MG were from ICLC and ECACC, respectively. CAS-1, HEK293T, LN-229, U-87MG and U-251MG were cultured in phenol red-free high glucose DMEM medium (Gibco, 31053028) supplemented with 10 % FBS (Gibco, 10270106), 2 mM L-glutamine (Gibco, 25030-024), 1 mM sodium pyruvate (Gibco, 11360-039), 100 U/mL penicillin and 100 µg/mL streptomycin (Gibco, 15140-122) (henceforth, referred to as complete DMEM. Cell lines were cultured at 37 °C and 5 % CO₂.

### Generation of transgenic cell lines

A human *IL4I1* cDNA clone flanked by Gateway compatible recombination sites was purchased from MyBiosource (MBS1270935). The cDNA clone was recombined into the lentivirus compatible Gateway expression vector pLX301 (a gift from D. Root, Addgene plasmid 25895) (Yang et al., 2011). Production of lentiviruses was achieved by transfecting HEK293T with pMD2.G (a gift from D. Trono, Addgene plasmid 12259), psPAX2 (a gift from D. Trono, Addgene plasmid 12260), and lentiviral plasmid, using FuGENE HD (Promega, E2311) according to the manufacturer's protocol. Viral supernatants were harvested at 48 h and 72 h, pooled and filtered through a 0.45 µm pore filter. Stable IL4I1-expressing (pLX301-IL4I1) and control (pLX301) cell lines were generated by infecting U-87MG and U-251MG cells for 24 h with the respective viral supernatants in presence of 8 µg/mL polybrene (Merck Millipore, TR-1003-G), followed by selection with medium containing 1 µg/mL puromycin (AppliChem, A2856). Stable expression of IL4I1 was confirmed by qRT-PCR, immunoblot and IL4I1 enzymatic activity.

Stable knockdown of AHR in U-87MG cells was achieved using shERWOOD UltramiR Lentiviral shRNA targeting AHR (transOMIC Technologies, TLHSU1400-196-GVO-TRI). Glioma cells were infected with viral supernatants containing either shAHR or shControl (shC) sequences to generate stable cell lines.
shERWOOD UltramiR shRNA sequences are:

shAHR (ULTRA-3234821): 5'-TGCTGTTGACAGTGAGCGCAGGAAGAATTGTTT
TAGGATATAGTGAAGCCACAGATGTATATCCTAAAACAATTCTTCCTTTGCC
TACTGCCTCGGA-3' (SEQ ID NO: 1);

shC (ULTRA-NT#4): 5'- TGCTGTTGACAGTGA GCG
AAGGCAGAAGTATGCAAAGCATTAGTGAAGCCACAGATGTAATGCTTTGCA
TACTTCTGCCTGTGCCTACTGCCTCGG A-3 (SEQ ID NO: 2)'.

siRNA mediated gene knockdown of *IL4I1* was carried out using ON-TARGETplus Human SMARTpool siRNA reagent (Dharmacon, L-008109-00-0005). siRNA transfections were done with Lipofectamine RNAiMAX (Thermo Fisher Scientific, 13778100), following the manufacturer's protocol. ON-TARGETplus Non-targeting Pool siRNA (Dharmacon, D-001810-10-05) was used as control.

For gene and protein expression experiments involving Ctrl and IL4I1-expressing U-87MG and U-251MG cells, 4 x 10⁵ cells per well were seeded in 2 mL in 6-well plates and incubated for 72 h or 120 h. For metabolomics experiments, cells were seeded at a density of 4 x 10⁵ cells per well in 2 mL of complete DMEM in 6-well plates and incubated for 24 h. In cases where more cells and supernatant were needed, 2.6 x 10⁶ cells per 10 cm dish were seeded in 13.3 mL of complete DMEM and incubated for 24 h. Afterwards, cells were washed once with phosphate-buffered saline (PBS), 2 or 13.3 mL of fresh FBS-free DMEM were added (depending on the well size and cell density used) and cells were incubated for 120 h. Supernatants were snap frozen in liquid nitrogen and stored at -80 °C until metabolite measurement.

Experiments targeting *AHR* with siRNA in U-87MG cells were achieved by seeding 4 x 10⁵ cells per well in 6-well plates, followed by incubation for 24 h. Cells were transfected with either siCtrl or siAHR and fresh medium containing treatment was added 48 h later. For experiments where *AHR* and *IL4I1* were knocked down in CAS-1 cells, 4 x 10⁵ cells per well in 6-well plates were seeded and incubated for 24 h. Cells were transfected with respective siCtrl or targeting siRNA. Complete DMEM was replaced with 1.5 mL of FBS-free DMEM 24 h post-transfection and cells were incubated for 72 h.

CAS-1 cells with stable downregulation of IL4I1 were generated using transEDIT CRISPR All-in-one lentiviral expression vectors targeting IL4I1 (transOMIC Technologies, CAHS1001-259307-GVO-TRI). Cells were exposed to viral supernatants containing either sgControl (sgCtrl) or sgIL4I1 for 24 h, in presence of 8 µg/mL polybrene (Merck Millipore, TR-1003-G). Transduced cells were selected by sorting ZsGreen positive cells using a BD FACSAria Fusion (BD Biosciences). transEDIT CRISPR All-in-one sgRNA sequences without PAM (5'-3') are:
sgCtrl (TELG1017): GGAGCGCACCATCTTCTTCA (SEQ ID NO: 27)
sgIL4I1-2 (TEVH-1143054): GGGCCGCATCTTCACCTACC (SEQ ID NO: 28)

### Migration assays

Cells were seeded in 2-well cell culture inserts (ibidi, 80209) placed in 24-well plates. For U-87MG cells, as well as Ctrl and IL4I1-expressing U-87MG cells, 3 x 10⁴ cells in 100 µL medium per well of cell culture insert were seeded. In the case of Ctrl and IL4I1-expressing U-251MG cells, 4 x 10⁴ in 100 µL medium per well of cell culture insert were seeded. Cells were incubated for 16 h, inserts were removed and 1 mL of complete DMEM medium was added to each well of the plate. Cell migration was monitored by taking pictures at indicated time points, until gap (500 µm +/- 100 µm) was covered by cells. In the case where U-87MG cells were treated with I3P or SR1, treatments were applied at the time of seeding the cells in the 2 well cell culture inserts. In addition, after 16 h incubation time and insert removal, 1 mL of complete DMEM medium containing treatments was applied to cells and kept throughout the assay. For each independent experiment, 3 pictures per well were taken and each condition was carried out at least in 3 wells. Pictures were taken with an ECLIPSE Ti-E/B inverted microscope (Nikon) with the 4 x objective and using the NIS Elements software version 4.13.04. Pictures were analyzed with TScratch software version 1.0 (Geback et al., 2009). Cell migration was assessed by calculating the percentage of covered area for each time point normalized to the area at the 0 h time point.

### RNA isolation and real time PCR

Total RNA was harvested from cultured cells using the RNeasy Mini Kit (Qiagen, 80204) followed by cDNA synthesis using the High Capacity cDNA reverse transcriptase kit (Applied Biosystems, 4368813). A StepOne Plus real-time PCR system (Applied Biosystems) was used to perform quantitative real-time PCR (qRT-PCR) of cDNA samples using SYBR Select Master mix (Thermo Scientific, 4309155). Data was processed and analyzed using the StepOne Software v2.3. Relative quantification of target genes was done against *RNA18S* as reference gene using the 2^{-ΔΔCt} method. Human primer sequences are listed in Table 3.

**Table 3 Human primer sequences**

| **Gene Name** | **Forward Primer (5'->3')** | **Reverse Primer (5'->3')** |
|---|---|---|
| *ABCG2* | TTCCACGATATGGATTTACGG (SEQ ID NO: 3) | GTTTCCTGTTGCATTGAGTCC (SEQ ID NO: 4) |
| *AHRR* | CCCTCCTCAGGTGGTGTTTG (SEQ ID NO: 5) | CGACAAATGAAGCAGCGTGT (SEQ ID NO: 6) |
| *CYP1B1* | GACGCCTTTATCCTCTCTGCG (SEQ ID NO: 7) | ACGACCTGATCCAATTCTGC C (SEQ ID NO: 8) |
| *EGR1* | CTGACCGCAGAGTCTTTTCCT (SEQ ID NO: 9) | GAGTGGTTTGGCTGGGGTAA (SEQ ID NO: 10) |
| *EREG* | CTGCCTGGGTTTCCATCTTCT (SEQ ID NO: 11) | GCCATTCATGTCAGAGCTAC ACT (SEQ ID NO: 12) |
| *IL1B* | CTCGCCAGTGAAATGATGGCT (SEQ ID NO: 13) | GTCGGAGATTCGTAGCTGGA T (SEQ ID NO: 14) |
| *IL4I1* | CGCCCGAAGACATCTACCAG (SEQ ID NO: 15) | GATATTCCAAGAGCGTGTGC C (SEQ ID NO: 16) |
| *MMP1* | GCTAACCTTTGATGCTATAACTA CGA (SEQ ID NO: 17) | TTTGTGCGCATGTAGAATCT G (SEQ ID NO: 18) |
| *NPTX1* | CATCAATGACAAGGTGGCCAAG (SEQ ID NO: 19) | GGGCTTGATGGGGTGATAGG (SEQ ID NO: 20) |
| *RNA18S* | GATGGGCGGCGGAAAATAG (SEQ ID NO: 21) | GCGTGGATTCTGCATAATGG T (SEQ ID NO: 22) |
| *SERPINB2* | ACCCCCATGACTCCAGAGAA (SEQ ID NO: 23) | CTTGTGCCTGCAAAATCGCA T (SEQ ID NO: 24) |
| *TIPARP* | CACCCTCTAGCAATGTCAACTC (SEQ ID NO: 25) | CAGACTCGGGATACTCTCTC C (SEQ ID NO: 26) |

### Protein Isolation and Western Blots

For all experiments, whole cell lysates were prepared in ice cold tris(hydroxymethyl)aminomethane (281 mM; Merck, 1083870500), Tris HCl (212 mM; Carl Roth, 9090.4) and EDTA (1 mM, pH 8; Gibco, 15575-038) containing 4 % sodium dodecyl sulfate (SDS; VWR, 442444H) and glycerol (40 %, Sigma Aldrich) supplemented with cOmplete™ Protease Inhibitor Cocktail (Roche, 11697498001). For AHR translocation assays, protein content in the nuclear and the cytoplasmic fractions of LN-229 glioblastoma cells was compared by immunoblotting. LN-229 cells were treated with supernatants of Ctrl and IL4I1-expressing U-251MG cells (120 h) for 4 h. Lysates were snap frozen in liquid nitrogen and thawed three times following 10 cycles of ultrasonication after each freeze-thaw cycle. To isolate protein from the two different cellular fractions NE-PER™ Nuclear and Cytoplasmic Extraction Reagents (Thermo Fisher Scientific, 78835) were used. Extraction was performed following the manufacturer's instructions. Nuclear specific Lamin A served as control for appropriate fractionation and was detected using polyclonal rabbit anti-Lamin A antibody (BioLegend, 613501; 1:500). AHR was detected using the primary mouse monoclonal anti-AHR antibody clone RPT1 (Abcam, ab2770). After incubation with the primary antibody membranes were washed three times for 10 min and incubated with a 1:5000 dilution of horseradish peroxidase-conjugated anti-rabbit (for IL4I1, Lamin A; BioRad, 170-6515) or anti-mouse antibody (for β-actin, AHR; BioRad, 170-6516) for 1 h at RT, respectively. Antibodies were prepared in wash buffer (50 mM Tris-HCL, 50 mM Tris-Base, 150 mM NaCl, pH 6.8) containing 2 % milk powder (Carl Roth, T145.2). Immunoblots were detected using ECL Select Western Blotting Detection Reagent (Amersham, RPN2235) on a FluorChem FC3 system (BioLabTec).

### Metabolomic analyses

Consumption of phenylalanine, tyrosine and tryptophan by control and IL4I1-expressing and non-expressing U-87MG and U-251MG cells was assessed by quantification of the amino acids in the cell culture supernatants after 120 h incubation. For phenylalanine and tyrosine detection, amino acids were labeled with the fluorescent dye AccQ-Tag™ (Waters) according to the manufacturer's protocol. The derivatization product was separated at 42°C on an Acquity BEH C18 column (Waters) using an Acquity H-class UPLC system (Waters) coupled to an Acquity fluorescence detector (FLR) (Waters). Samples were analyzed on the UPLC as described by Yang et al. (2015) (Yang et al., 2015). For analyses of Trp consumption, supernatants were mixed with an equal volume of 12 % perchloric acid and incubated on ice for 10 min. Prior to analysis, samples were centrifuged for 10 min at 4°C and 16,400 g to precipitate proteins and remove remaining cell debris. Metabolites were then separated by reversed phase chromatography on an Acquity HSS T3 column (100 mm x 2.1 mm, 1.7 µm, Waters) connected to an Acquity H-class UPLC system (Waters). The column was heated to 37 °C and equilibrated with 5 column volumes of 100 % solvent A (20 mM sodium acetate, 3 mM zinc acetate, pH 6) at a flow rate of 0.55 mL/min. Clear separation of Trp was achieved by increasing the concentration of solvent B (acetonitrile) in solvent A as follows: 4 min 0 % B, 10 min 5 % B, 13 min 15 % B, 15 min 25 % B, and return to 0 % B in 3 min. Trp was detected by fluorescence (Acquity FLR detector, Waters, excitation: 254 nm, emission: 401 nm). Standards were used for quantification (Sigma). Data acquisition and processing was performed with the Empower3 software suite (Waters).

Further, the inventors conducted an untargeted metabolomics approach to identify metabolites that were differentially abundant in supernatants of Ctrl and IL4I1-expressing U-87MG and U-251MG cells cultured for 120 h. To this end, 50 µL of cell culture supernatants were mixed with 200 µL ice-cold acetonitrile by vortexing followed by incubation at -20 °C for 1 h. Samples were centrifuged for 15 min at 4 °C and transferred into TruView UPLC-MS vials (Waters). A pool sample was prepared by mixing equal volumes of all samples. Samples were measured using an I-class UPLC system coupled to a Vion IMS QTof MS (Waters). Metabolites were separated using either a Cogent Diamond Hydride 2.0 column (150 x 2.1 mm, 2.2 µm; MicroSolv USA) or a HSS T3 column (100 x 2.1 mm, 1.8 µm; Waters). For instrument control and acquisition of MS data UNIFI 1.8.2 (Waters) was used. Follow-up data analyses were performed using Progenesis QI (Waters). ILA was detected at 204.0662 Da (neg. mode; expected monoisotopic mass: 205.0739 Da; deviation -2 ppm) and identified by the expected fragment ions at 116.0495, 128.0495, 130.0652 and 204.0655 Da. The Trp-, Phe- and Tyr-derived metabolites were identified by comparing their fragmentation patterns resulting from LC-MS measurements using an HPLC (Agilent 1290) coupled to a triple quad MS (Agilent 6460) with external standards. For targeted quantification of the metabolites, MRM mode was used. For all test compounds 10 mM stock solutions were prepared by gravimetrically adding the required amount into 1.5 mL Eppendorf safe-lock tubes and dissolving the test compound in 1 mL DMSO. For each compound, stock solutions covered a concentration range from 10 mM to 0.039 mM.

For quantification of the metabolites, 300 µL of each bioassay supernatant was added to Eppendorf safe-lock tubes. Associated calibration samples were prepared by adding 300 µL cell culture media and 1 µL of test compound stock solution into an Eppendorf safe-lock tube. Subsequently, 300 µL acetonitrile was added to trigger precipitation of media components. All samples were centrifuged at 8000 rpm for 4 min and 150 µL of the supernatants was transferred into 1.5 mL glass vials equipped with 200 µL glass inserts for HPLC analysis. 5 µL of the sample was injected for the analysis. Water and acetonitrile with 0.1 % formic acid were used as eluent A and B, respectively for HPLC separation for 5 min with a flow rate of 0.5 mL/min. Separation was achieved using a 50 mm long Poroshell 120 EC-C18 2.7 micron column (Agilent). The Agilent Jetstream ESI source was set to gas with a gas sheath temperature of 300 °C, a gas flow of 10 L/min and a sheath gas flow of 11 L/min. The nebulizer pressure was set to 55 psi and capillary voltage at 2000 V throughout the run. For instrument control and data acquisition MassHunter software suite (Agilent) was used.

### Software and statistics

Graphical and statistical analyses of qRT-PCR, protein, as well as metabolite data were performed using GraphPad Prism software version 8.0. Unless otherwise indicated, data represents the mean ± S.E.M of at least 3 independent experiments. In cases where data was expressed as fold change, these values were log10 transformed and the resulting values were used for statistical analysis. Depending on the data, the following statistical analyses were applied: one sample *t*-test, two-tailed student's *t*-test (paired or unpaired), one-way ANOVA with Tukey's multiple comparisons test and repeated measures ANOVA with Dunnett's multiple comparisons test. Significant differences were reported as * *P* < 0.05, ** *P* < 0.01, *** *P* < 0.001, **** *P* < 0.0001. n.s. indicates no significant difference. For bioinformatics analysis, unless stated otherwise, all pairwise comparisons were performed using Kruskal-Wallis and Wilcoxon sum rank tests, and all reported p-values were adjusted using the Benjamini-Hochberg procedure.

The L-amino acid oxidase IL4I1 exhibited the highest incidence in AHR associated modules (AAMs), as it was present in 26 out of the 32 tumor types (Figure 1). Thus, AHR activity in human tumors associates more strongly with IL4I1 than with any other Trp-degrading enzyme, including the two *bona fide* AHR activators *IDO1* and *TDO2.* This was surprising, as IL4I1 is mainly recognized for catalyzing the oxidative deamination of phenylalanine (Phe) to phenylpyruvate (PP), while producing hydrogen peroxide (H₂O₂) and ammonia (Boulland et al., 2007; Mason et al., 2004), but has not yet been implicated in AHR signaling.

IL4I1 activates the AHR. The inventors next tested experimentally whether IL4I1 activates the AHR. For this purpose, the inventors opted for human cell cultures and tissues, as mice express an additional L-amino acid oxidase (*Lao1*) (Hughes, 2010) that can compensate for *Il4i1* deficiency. IL4I1 protein was expressed in human GBM tissue (Figure 2A), suggesting GBM cells as a suitable model to investigate IL4I1. The inventors compared AHR-proficient (U87-MG) with AHR-deficient (U-251MG) GBM cell lines. IL4I1 was ectopically expressed in cell lines with low endogenous IL4I1, or downregulated by RNA interference or CRISPR/Cas9 in a cell line with high endogenous IL4I1. Ectopic expression of IL4I1 enhanced its enzymatic activity, as assessed by H₂O₂ levels. In the AHR-proficient cells, IL4I1 induced the pan-tissue AHR signature (Figure 2B, top), which was reversed by AHR knockdown (Figures 2B, bottom). Selected AHR target genes were confirmed by qRT-PCR (Figure 2C). To test whether IL4I1-derived metabolites activate the AHR, the inventors treated GBM cells with supernatants of cells with ectopic IL4I1. In support of the inventors' hypothesis, the supernatants elicited increased nuclear AHR localization (Figure 2D) and target gene transcription (Figure 2E). Conversely, IL4I1 downregulation decreased expression of the AHR target genes *IL1B, EREG, SERPINB2* and *TIPARP* (Figure 2F).

IL4I1 promotes tumor cell motility, suppresses T cell proliferation, and is associated with reduced survival in glioma patients. To test IL4I1's contribution to the functional outcomes of the AHR, the inventors first investigated the role of IL4I1 in GBM cell motility. Ectopic IL4I1 increased the motility of AHR-proficient cells (Figure 3A), but not of AHR-deficient cells (Figure 3B). In keeping with this result, the IL4I1-mediated motility of AHR-proficient cells was reversed by the AHR inhibitor SR1 (Figure 3C).

To translate the inventors' findings to patients, the inventors analysed the association of the three AHR-activating enzymes IL4I1, IDOl, and TDO2, with overall survival probability in GBM. High *IL4I1* levels associated with reduced overall survival (Figure 3D). In fact, GBM patients with high tumoral *IL4I1* expression had a 2.3 times higher risk of death compared to patients with low *IL4I1* expression (Figure 3D, and Table 2).

High *TDO2* levels associated with a 1.5 times higher risk of death, while no significant survival difference was associated with *IDOl* transcript levels (Figure 3E and Table S6).

Similarly, low-grade glioma (LGG) with high expression of *IL4I1, IDO1* and *TDO2* were all associated with a higher risk of death (Figure 3E). The risk increase was highest for *IL4I1* expression (3.3 fold), followed by *IDOl* (2.4 fold), and *TDO2* (1.6 fold) (Figure 3E, and Table 2).

Indole-3-pyruvate is the key metabolite mediating IL4I1-driven AHR activity and malignancy. The inventors next asked which IL4I1-derived metabolites activate the AHR. In keeping with previous reports (Boulland et al., 2007; Mason et al., 2004), IL4I1 reduced the levels of Phe, tyrosine (Tyr), and Trp in cell culture supernatants (Figure 4A). IL4I1 converts Phe, Tyr, and Trp to PP, hydroxyphenylpyruvic acid (HPP), and indole-3-pyruvic acid (I3P), respectively. In agreement with its AHR-activating properties, I3P enhanced GBM cell motility in an AHR-dependent manner (Figure 4B-C). I3P hence represents a novel oncometabolite that mediates IL4I1 and AHR-driven malignant properties.

Indole-3-pyruvate gives rise to the AHR agonists kynurenic acid and indole-3-aldehyde. Surprisingly, all known IL4I1 products but I3P were detectable in supernatants of cells with ectopic IL4I1 (Figures 5 and 6). Yet, the inventors detected increased levels of I3P derivatives, including indole-3-acetic acid (IAA), indole-3-aldehyde (I3A) and indole-3-lactic acid (ILA) (Figures 5A and 6C). Moreover, IL4I1 enhanced the levels of KynA, while it did not affect Kyn levels (Figures 5A and 6C). KynA formation from I3P was unexpected as KynA is typically considered to originate from IDO1- or TDO2-derived Kyn (Cervenka et al., 2017; Platten et al., 2019). One reaction through which I3P could enhance KynA levels is the transamination of Kyn, as kynurenine aminotransferase (KAT) can use alpha-ketoacids as amino group acceptors (Han et al., 2004). However, Kyn concentrations were not reduced in cells with ectopic IL4I1 (Figures 5 and 6C), rendering this hypothesis unlikely. Interestingly, KynA formed spontaneously from I3P under cell-free conditions (data not shown). This was enhanced in the presence of H₂O₂ (data not shown), suggesting that IL4I1-derived H₂O₂ promotes I3P's conversion to the AHR agonist KynA.

IL4I1 expression is increased in cancer and metastasis. To explore IL4I1's potential as a therapeutic target in malignancies the inventors investigated its expression in normal tissues versus primary tumors and metastases. Across most tumor entities, *IL4I1* expression was enhanced in primary cancer tissues compared to normal tissues (Figures 7A and 7B). In the majority of tumors, *IL4I1* expression was higher than *IDOl* or *TDO2,* highlighting IL4I1 as a major Trp-catabolizing enzyme in cancer (Figure 7B). In support of their malignant properties, *IL4I1* levels (Figure 7C) and AHR activity (Figure 7D) were significantly higher in metastatic melanoma, as compared to primary melanoma. In agreement, IL4I1 enzymatic activity was detected in freshly excised metastatic melanoma tissue (Figure 7E).

Although IL4I1 drives motility and metastasis, *motility* was only the third most enriched ontology group for AAMs containing IL4I1 (Figure 7F). In fact, immune modulation was most enriched, and thus might be an even more prominent IL4I1 outcome in cancer. Across the 32 cancer types, *IL4I1* high expressing tumors showed an enrichment of suppressive immune cells such as myeloid-derived suppressor cells (MDSCs) and Treg cells (Figures 8A and 8B).

ICB through antibodies targeting the PD-1/ (programmed cell death 1 ligand 1) PD-L1 interaction has been approved for multiple cancers based on unprecedented clinical responses in a fraction of patients. However, most cancer patients do not benefit from ICB, and there is an urgent need to identify the molecular basis for ICB resistance. IL4I1's association with suppressive immune cells suggests that IL4I1 could circumvent ICB by activating an alternative immunosuppressive mechanism. Therefore, the inventors investigated whether Trp-degrading enzymes are upregulated upon ICB. The inventors found that the anti-PD-1 monoclonal antibody (mAb) nivolumab induced IL4I1 and IDOl expression in advanced melanoma (Figure 8C) (Riaz et al., 2017). Moreover, nivolumab treatment resulted in AHR activation (Figure 8D), suggesting that IL4I1 signaling to the AHR represents a novel metabolic immune checkpoint that mediates ICB resistance. To further investigate this possibility, the inventors analyzed complete response (CR) information of matched before and on treatment RNA-seq data from 41 patients. Out of these, 24 patients had received the anti-CTLA4 mAb ipilimumab, prior to nivolumab initiation (Riaz et al., 2017). In agreement with the entire cohort (Figure 8C), the inventors observed a significant IL4I1 increase in response to nivolumab in the ipilimumab-naïve patients (Figure 8E). Of note, *IL4I1* (but not *IDO1*) and immune checkpoint (CP) molecules were significantly induced in ipilimumab-naive patients that developed progressive disease (PD) (Figure 8F,), suggesting that IL4I1 induction represents a resistance mechanism against ICB.

Based on the notion that IDO1 might constitute a potential escape mechanism from anti-PD-1 ICB, a recent phase III clinical trial in advanced melanoma tested the clinical benefit of the IDOl inhibitor epacadostat in combination with the anti-PD-1 mAb pembrolizumab (Long et al., 2019). However, this trial failed (Garber, 2018; Long et al., 2019; Muller et al., 2019; Platten et al., 2019). The inventors hypothesized that in light of their downstream convergence on the AHR, IL4I1 could constitute a resistance mechanism against IDO1 inhibition. The inventors propose that by activating the AHR, IL4I1 represents a resistance mechanism against ICB and/or IDOl inhibitors. IL4I1 therefore is a promising target for tumor therapy.

### References as cited

Agudelo, L.Z., et al. (2018). Kynurenic Acid and Gpr35 Regulate Adipose Tissue Energy Homeostasis and Inflammation. Cell metabolism 27, 378-392 e375.
Aoki, R., et al. (2018). Indole-3-Pyruvic Acid, an Aryl Hydrocarbon Receptor Activator, Suppresses Experimental Colitis in Mice. J Immunol 201, 3683-3693.
Argelaguet, R., et al. (2018). Multi-Omics Factor Analysis-a framework for unsupervised integration of multi-omics data sets. Mol Syst Biol 14, e8124.
Bittinger, M.A., Nguyen, L.P., and Bradfield, C.A. (2003). Aspartate aminotransferase generates proagonists of the aryl hydrocarbon receptor. Mol Pharmacol 64, 550-556.
Boitano, A.E., et al. (2010). Aryl hydrocarbon receptor antagonists promote the expansion of human hematopoietic stem cells. Science 329, 1345-1348.
Boulland, M.L., M et al. (2007). Human IL4I1 is a secreted L-phenylalanine oxidase expressed by mature dendritic cells that inhibits T-lymphocyte proliferation. Blood 110, 220-227.
Boyle, E.I., Weng, S., Gollub, J., Jin, H., Botstein, D., Cherry, J.M., and Sherlock, G. (2004). GO::TermFinder--open source software for accessing Gene Ontology information and finding significantly enriched Gene Ontology terms associated with a list of genes. Bioinformatics 20, 3710-3715.
Brennan, J.C., et al. (2015). Development of Species-Specific Ah Receptor-Responsive Third Generation CALUX Cell Lines with Enhanced Responsiveness and Improved Detection Limits. Environ Sci Technol 49, 11903-11912.
Bui, Q.C., and Sloot, P.M. (2012). A robust approach to extract biomedical events from literature. Bioinformatics 28, 2654-2661.
Carbonnelle-Puscian, A.,, et al. (2009). The novel immunosuppressive enzyme IL4I1 is expressed by neoplastic cells of several B-cell lymphomas and by tumor-associated macrophages. Leukemia 23, 952-960.
Carvalho, B., Bengtsson, H., Speed, T.P., and Irizarry, R.A. (2007). Exploration, normalization, and genotype calls of high-density oligonucleotide SNP array data. Biostatistics 8, 485-499.
Cervantes-Barragan, L., et al. (2017). Lactobacillus reuteri induces gut intraepithelial CD4(+)CD8alphaalpha(+) T cells. Science 357, 806-810.
Cervenka, I., Agudelo, L.Z., and Ruas, J.L. (2017). Kynurenines: Tryptophan's metabolites in exercise, inflammation, and mental health. Science 357.
Charoentong, P., Finotello, F., Angelova, M., Mayer, C., Efremova, M., Rieder, D., Hackl, H., and Trajanoski, Z. (2017). Pan-cancer Immunogenomic Analyses Reveal Genotype-Immunophenotype Relationships and Predictors of Response to Checkpoint Blockade. Cell Rep 18, 248-262.
Chen, J.Y., Li, C.F., Kuo, C.C., Tsai, K.K., Hou, M.F., and Hung, W.C. (2014). Cancer/stroma interplay via cyclooxygenase-2 and indoleamine 2,3-dioxygenase promotes breast cancer progression. Breast cancer research : BCR 16, 410.
Colaprico, A., et al. (2016). TCGAbiolinks: an R/Bioconductor package for integrative analysis of TCGA data. Nucleic Acids Res 44, e71.
D'Amato, N.C., et al. (2015). A TDO2-AhR signaling axis facilitates anoikis resistance and metastasis in triple-negative breast cancer. Cancer Res 75, 4651-4664.
Davis, S., and Meltzer, P.S. (2007). GEOquery: a bridge between the Gene Expression Omnibus (GEO) and BioConductor. Bioinformatics 23, 1846-1847.
Denison, M.S., and Nagy, S.R. (2003). Activation of the aryl hydrocarbon receptor by structurally diverse exogenous and endogenous chemicals. Annu Rev Pharmacol Toxicol 43, 309-334.
DiNatale, B.C., et al. (2010). Kynurenic acid is a potent endogenous aryl hydrocarbon receptor ligand that synergistically induces interleukin-6 in the presence of inflammatory signaling. Toxicol Sci 115, 89-97.
Dodd, D., et al. (2017). A gut bacterial pathway metabolizes aromatic amino acids into nine circulating metabolites. Nature 551, 648-652.
Du, P., Kibbe, W.A., and Lin, S.M. (2008). lumi: a pipeline for processing Illumina microarray. Bioinformatics 24, 1547-1548.
Edgar, R., Domrachev, M., and Lash, A.E. (2002). Gene Expression Omnibus: NCBI gene expression and hybridization array data repository. Nucleic Acids Res 30, 207-210.
Esser, C., Rannug, A., and Stockinger, B. (2009). The aryl hydrocarbon receptor in immunity. Trends Immunol 30, 447-454.
Fernandez-Salguero, P., et al. (1995). Immune system impairment and hepatic fibrosis in mice lacking the dioxin-binding Ah receptor. Science 268, 722-726.
Fong, M.Y., McDunn, J., and Kakar, S.S. (2011). Identification of metabolites in the normal ovary and their transformation in primary and metastatic ovarian cancer. PLoS ONE 6, 1-12.
Gabriely, G., Wheeler, M.A., Takenaka, M.C., and Quintana, F.J. (2017). Role of AHR and HIF-1alpha in Glioblastoma Metabolism. Trends Endocrinol Metab 28, 428-436.
Gagliani, N., et al. (2015). Th17 cells transdifferentiate into regulatory T cells during resolution of inflammation. Nature 523, 221-225.
Garber, K. (2018). A new cancer immunotherapy suffers a setback. Science 360, 588.
Gautier, L., Cope, L., Bolstad, B.M., and Irizarry, R.A. (2004). affy--analysis of Affymetrix GeneChip data at the probe level. Bioinformatics 20, 307-315.
Geback, T., Schulz, M.M., Koumoutsakos, P., and Detmar, M. (2009). TScratch: a novel and simple software tool for automated analysis of monolayer wound healing assays. Biotechniques 46, 265-274.
Goeman, J.J., and Finos, L. (2012). The inheritance procedure: multiple testing of tree-structured hypotheses. Stat Appl Genet Mol Biol 11, Article 11.
Goeman, J.J., van de Geer, S.A., de Kort, F., and van Houwelingen, H.C. (2004). A global test for groups of genes: testing association with a clinical outcome. Bioinformatics 20, 93-99.
Greene, L.I., Bruno, T.C., Christenson, J.L., D'Alessandro, A., Culp-Hill, R., Torkko, K., Borges, V.F., Slansky, J.E., and Richer, J.K. (2019). A role for tryptophan-2,3-dioxygenase in CD8 T-cell suppression and evidence of tryptophan catabolism in breast cancer patient plasma. Molecular Cancer Research 17, 131-139.
Gu, Z., Eils, R., and Schlesner, M. (2016). Complex heatmaps reveal patterns and correlations in multidimensional genomic data. Bioinformatics 32, 2847-2849.
Gu, Z., Gu, L., Eils, R., Schlesner, M., and Brors, B. (2014). circlize Implements and enhances circular visualization in R. Bioinformatics 30, 2811-2812.
Gutierrez-Vazquez, C., and Quintana, F.J. (2018). Regulation of the Immune Response by the Aryl Hydrocarbon Receptor. Immunity 48, 19-33.
Han, Q., Li, J., and Li, J. (2004). pH dependence, substrate specificity and inhibition of human kynurenine aminotransferase I. Eur J Biochem 271, 4804-4814.
Hanzelmann, S., Castelo, R., and Guinney, J. (2013). GSVA: gene set variation analysis for microarray and RNA-seq data. BMC bioinformatics 14, 7.
Hothorn, T., and Zeileis, A. (2008). Generalized maximally selected statistics. Biometrics 64, 1263-1269.
Huang, J., et al. (2016). Serum metabolomic profiling of prostate cancer risk in the prostate, lung, colorectal, and ovarian cancer screening trial. British journal of cancer 115, 1087-1095.
Hubbard, T.D., Murray, I.A., and Perdew, G.H. (2015). Indole and Tryptophan Metabolism: Endogenous and Dietary Routes to Ah Receptor Activation. Drug Metab Dispos 43, 1522-1535.
Hughes, A.L. (2010). Origin and diversification of the L-amino oxidase family in innate immune defenses of animals. Immunogenetics 62, 753-759.
Kanehisa, M., and Goto, S. (2000). KEGG: kyoto encyclopedia of genes and genomes. Nucleic Acids Res 28, 27-30.
Kiss, E.A., Vonarbourg, C., Kopfmann, S., Hobeika, E., Finke, D., Esser, C., and Diefenbach, A. (2011). Natural aryl hydrocarbon receptor ligands control organogenesis of intestinal lymphoid follicles. Science 334, 1561-1565.
Langfelder, P., and Horvath, S. (2008). WGCNA: an R package for weighted correlation network analysis. BMC bioinformatics 9, 559.
Lasoudris, F., Cousin, C., Prevost-Blondel, A., Martin-Garcia, N., Abd-Alsamad, I., Ortonne, N., Farcet, J-P., Castellano F., Molinier-Frenkel, V. (2011). IL4I1: an inhibitor of the CD8+ antitumor T-cell response in vivo, Eur J Immunol. Jun;41(6):1629-38)
Law, C.W., Alhamdoosh, M., Su, S., Dong, X., Tian, L., Smyth, G.K., and Ritchie, M.E. (2016). RNA-seq analysis is easy as 1-2-3 with limma, Glimma and edgeR. F1000Res 5.
Law, C.W., Chen, Y., Shi, W., and Smyth, G.K. (2014). voom: Precision weights unlock linear model analysis tools for RNA-seq read counts. Genome biology 15, R29.
Lemieux, G.A., Cunningham, K.A., Lin, L., Mayer, F., Werb, Z., and Ashrafi, K. (2015). Kynurenic acid is a nutritional cue that enables behavioral plasticity. Cell 160, 119-131.
Lemos, H., Huang, L., Prendergast, G.C., and Mellor, A.L. (2019). Immune control by amino acid catabolism during tumorigenesis and therapy. Nature Reviews Cancer 19, 162-175.
Li, B., and Dewey, C.N. (2011). RSEM: accurate transcript quantification from RNA-Seq data with or without a reference genome. BMC bioinformatics 12, 323.
Li, Y., et al. (2011). Exogenous stimuli maintain intraepithelial lymphocytes via aryl hydrocarbon receptor activation. Cell 147, 629-640.
Lin, S.M., Du, P., Huber, W., and Kibbe, W.A. (2008). Model-based variance-stabilizing transformation for Illumina microarray data. Nucleic Acids Res 36, e11.
Liu, Y., et al. (2018). Tumor-Repopulating Cells Induce PD-1 Expression in CD8(+) T Cells by Transferring Kynurenine and AhR Activation. Cancer cell 33, 480-494.e487.
Locasale, J.W., et al. (2012). Metabolomics of human cerebrospinal fluid identifies signatures of malignant glioma. Molecular and Cellular Proteomics 11, 1-12.
Long, G.V., et al. (2019). Epacadostat plus pembrolizumab versus placebo plus pembrolizumab in patients with unresectable or metastatic melanoma (ECHO-301/KEYNOTE-252): a phase 3, randomised, double-blind study. The Lancet Oncology 20, 1083-1097.
Marshall, N.B., and Kerkvliet, N.I. (2010). Dioxin and immune regulation: emerging role of aryl hydrocarbon receptor in the generation of regulatory T cells. Annals of the New York Academy of Sciences 1183, 25-37.
Mason, J.M., Naidu, M.D., Barcia, M., Porti, D., Chavan, S.S., and Chu, C.C. (2004). IL-4-induced gene-1 is a leukocyte L-amino acid oxidase with an unusual acidic pH preference and lysosomal localization. J Immunol 173, 4561-4567.
Mayer, A.K., et al. (2019). Homozygous stop mutation in AHR causes autosomal recessive foveal hypoplasia and infantile nystagmus. Brain 142, 1528-1534.
McGettrick, A.F., et al. (2016). Trypanosoma brucei metabolite indolepyruvate decreases HIF-1α and glycolysis in macrophages as a mechanism of innate immune evasion. Proceedings of the National Academy of Sciences of the United States of America 113, E7778-E7787.
Mezrich, J.D., Fechner, J.H., Zhang, X., Johnson, B.P., Burlingham, W.J., and Bradfield, C.A. (2010). An Interaction between Kynurenine and the Aryl Hydrocarbon Receptor Can Generate Regulatory T Cells. J Immunol 185, 3190-3198.
Molinier-Frenkel, V., Prevost-Blondel, A., and Castellano, F. (2019). The IL4I1 Enzyme: A New Player in the Immunosuppressive Tumor Microenvironment. Cells 8*.*
Muller, A.J., Manfredi, M.G., Zakharia, Y., and Prendergast, G.C. (2019). Inhibiting IDO pathways to treat cancer: lessons from the ECHO-301 trial and beyond. Semin Immunopathol 41, 41-48.
Murray, I.A., Patterson, A.D., and Perdew, G.H. (2014). Aryl hydrocarbon receptor ligands in cancer: Friend and foe. Nature Reviews Cancer 14, 801-814.
Natividad, J.M., et al. (2018). Impaired Aryl Hydrocarbon Receptor Ligand Production by the Gut Microbiota Is a Key Factor in Metabolic Syndrome. Cell metabolism 28, 737-749 e734.
Neale, P.A., et al. (2017). Development of a bioanalytical test battery for water quality monitoring: Fingerprinting identified micropollutants and their contribution to effects in surface water. Water Res 123, 734-750.
Neamah, W.H., et al. (2019). AhR Activation Leads to Massive Mobilization of Myeloid-Derived Suppressor Cells with Immunosuppressive Activity through Regulation of CXCR2 and MicroRNA miR-150-5p and miR-543-3p That Target AntiInflammatory Genes. The Journal of Immunology, ji1900291.
Nguyen, L.P., and Bradfield, C.A. (2008). The search for endogenous activators of the aryl hydrocarbon receptor. Chem Res Toxicol 21, 102-116.
Novikov, O., et al. (2016). An Aryl Hydrocarbon Receptor-Mediated Amplification Loop That Enforces Cell Migration in ER-/PR-/Her2- Human Breast Cancer Cells. Mol Pharmacol 90, 674-688.
Opitz, C.A., et al. (2011). An endogenous tumour-promoting ligand of the human aryl hydrocarbon receptor. Nature 478, 197-203.
Phipson, B., Lee, S., Majewski, I.J., Alexander, W.S., and Smyth, G.K. (2016). Robust Hyperparameter Estimation Protects against Hypervariable Genes and Improves Power to Detect Differential Expression. Ann Appl Stat 10, 946-963.
Plaisier, C.L., et al. (2016). Causal Mechanistic Regulatory Network for Glioblastoma Deciphered Using Systems Genetics Network Analysis. Cell systems 3, 172-186.
Platten, M., Nollen, E.A.A., Rohrig, U.F., Fallarino, F., and Opitz, C.A. (2019). Tryptophan metabolism as a common therapeutic target in cancer, neurodegeneration and beyond. Nature reviews Drug discovery.
Politi, V., Lavaggi, M.V., Di Stazio, G., and Margonelli, A. (1991). Indole-3-pyruvic acid as a direct precursor of kynurenic acid. Adv Exp Med Biol 294, 515-518.
Psachoulia, K., Chamberlain, K.A., Heo, D., Davis, S.E., Paskus, J.D., Nanescu, S.E., Dupree, J.L., Wynn, T.A., and Huang, J.K. (2016). IL4I1 augments CNS remyelination and axonal protection by modulating T cell driven inflammation. Brain 139, 3121-3136.
Quintana, F.J., Basso, A.S., Iglesias, A.H., Korn, T., Farez, M.F., Bettelli, E., Caccamo, M., Oukka, M., and Weiner, H.L. (2008). Control of T(reg) and T(H)17 cell differentiation by the aryl hydrocarbon receptor. Nature 453, 65-71.
Quintana, F.J., Murugaiyan, G., Farez, M.F., Mitsdoerffer, M., Tukpah, A.M., Burns, E.J., and Weiner, H.L. (2010). An endogenous aryl hydrocarbon receptor ligand acts on dendritic cells and T cells to suppress experimental autoimmune encephalomyelitis. Proc Natl Acad Sci U S A 107, 20768-20773.
Rentas, S., et al. (2016). Musashi-2 attenuates AHR signalling to expand human haematopoietic stem cells. Nature 532, 508-511.
Riaz, N., et al. (2017). Tumor and Microenvironment Evolution during Immunotherapy with Nivolumab. Cell 171, 934-949 e916.
Ritchie, M.E., Phipson, B., Wu, D., Hu, Y., Law, C.W., Shi, W., and Smyth, G.K. (2015). limma powers differential expression analyses for RNA-sequencing and microarray studies. Nucleic Acids Res 43, e47.
Robinson, M.D., McCarthy, D.J., and Smyth, G.K. (2010). edgeR: a Bioconductor package for differential expression analysis of digital gene expression data. Bioinformatics 26, 139-140.
Robinson, M.D., and Oshlack, A. (2010). A scaling normalization method for differential expression analysis of RNA-seq data. Genome biology 11, R25.
Rothhammer, V., et al. (2018). Microglial control of astrocytes in response to microbial metabolites. Nature 557, 724-728.
Rothhammer, V., and Quintana, F.J. (2019). The aryl hydrocarbon receptor: an environmental sensor integrating immune responses in health and disease. Nat Rev Immunol.
Russi, P., Carla, V., and Moroni, F. (1989). Indolpyruvic acid administration increases the brain content of kynurenic acid. Is this a new avenue to modulate excitatory amino acid receptors in vivo? Biochem Pharmacol 38, 2405-2409.
Schwarcz, R., Bruno, J.P., Muchowski, P.J., and Wu, H.Q. (2012). Kynurenines in the mammalian brain: when physiology meets pathology. Nature reviews Neuroscience 13, 465-477.
Smirnova, A., et al. (2016). Evidence for New Light-Independent Pathways for Generation of the Endogenous Aryl Hydrocarbon Receptor Agonist FICZ. Chemical research in toxicology 29, 75-86.
Stockinger, B., Di Meglio, P., Gialitakis, M., and Duarte, J.H. (2014). The aryl hydrocarbon receptor: multitasking in the immune system. Annu Rev Immunol 32, 403-432.
Stone, T.W., Stoy, N., and Darlington, L.G. (2013). An expanding range of targets for kynurenine metabolites of tryptophan. Trends Pharmacol Sci 34, 136-143.
Takenaka, M.C., et al. (2019). Control of tumor-associated macrophages and T cells in glioblastoma via AHR and CD39. Nature neuroscience 22, 729-740.
Wang, G.L., Jiang, B.H., Rue, E.A., and Semenza, G.L. (1995). Hypoxia-inducible factor 1 is a basic-helix-loop-helix-PAS heterodimer regulated by cellular O2 tension. Proc Natl Acad Sci U S A 92, 5510-5514.
Wermter, J., Tomanek, K., and Hahn, U. (2009). High-performance gene name normalization with GeNo. Bioinformatics 25, 815-821.
Wincent, E., et al. (2012). Inhibition of cytochrome P4501-dependent clearance of the endogenous agonist FICZ as a mechanism for activation of the aryl hydrocarbon receptor. Proc Natl Acad Sci U S A 109, 4479-4484.
Wu, D., et al.. (2010). ROAST: rotation gene set tests for complex microarray experiments. Bioinformatics 26, 2176-2182.
Xiang, Z., et al. (2019). A positive feedback between IDOl metabolite and COL12A1 via MAPK pathway to promote gastric cancer metastasis. J Exp Clin Cancer Res 38, 314.
Yang, X., et al. (2011). A public genome-scale lentiviral expression library of human ORFs. Nat Methods 8, 659-661.
Yang, Y., et al. (2015). Relation between chemotaxis and consumption of amino acids in bacteria. Mol Microbiol 96, 1272-1282.
Yu, G., et al. (2010). GOSemSim: an R package for measuring semantic similarity among GO terms and gene products. Bioinformatics 26, 976-978.
Yu, G., Wang, L.G., Han, Y., and He, Q.Y. (2012). clusterProfiler: an R package for comparing biological themes among gene clusters. OMICS 16, 284-287.
Zelante, T., et al. (2013). Tryptophan catabolites from microbiota engage aryl hydrocarbon receptor and balance mucosal reactivity via interleukin-22. Immunity 39, 372-385.

## Claims

1. A method for detecting and/or diagnosing cancer in a patient comprising detecting the change in IL4I1 expression and/or enzymatic activity of II,4I1 in a sample obtained from said patient, and diagnosing and/or detecting cancer in said patient, if said expression or enzymatic activity of said IL4I1 is increased in said patient when compared to control genes e.g, one or more housekeeping genes in said sample, or a control sample, e.g. a sample derived from a healthy subject, a group of healthy subjects, a patient or patient.

2. The method according to claim 1, wherein said detecting enzymatic activity of IL4I1 in said sample comprises the detection of the amount and/or concentration of II,4I1 metabolites in said sample.

3. The method according to claim 2, wherein said metabolites are selected from metabolites derived from the conversion of IL4I1 of phenylalanine, tyrosine and/or tryptophan, such as, for example, phenylpyruvic acid (PP), hydroxyphenylpyruvic acid (HPP), indole-3-pyruvic acid (I3P), 2-phenylacetic acid, phenyllactic acid, 4-hydroxybenzaldehyde, 2-hydroxy-2-phenylacetic acid, 4-hydroxyphenyllactic acid, and in particular the I3P derivatives indole-3-acetic acid (IAA), indole-3-aldehyde (I3A) and indole-3-lactic acid (ILA), 4-hydroxyquinoline-2-carboxylic acid (KynA), 1,3-di(IH-indole-3-yl)acetone, (3*Z*)-1-(1*H*-indole-3-yl)-3 -indole-3 -ylidenepropan-2-one, indole-3 -carboxylic acid, oxidized indole-3-acetic acid, and indole-3-carbinol and/or the amino acids or amino acid metabolites L-valine, L-isoleucine, L-leucine, L-alanine, L-glutamic acid, L-methionine, L-glutamine, 4-methylsulfanyl-2-oxobutanoate, alpha-keto-isoleucine, alpha-ketoisovalerate, alpha-ketoisocaproic acid, L-proline, and alpha-ketoglutaric acid, and any of the above in combination with ammonia and/or H₂O₂.

4. The method according to any one of claims 1 to 3, wherein detecting the enzymatic activity of IL4I1 comprises the use of chromatography NMR, metabolite sensors, antibodies, ELISAs, enzymatic assays, colorimetric assays, fluorescence assays or H₂O₂ or ammonia detection, and/or detecting expression using genetic tools, such as chip analysis and primers and probes and PCR analysis, or detecting the amount of protein of IL4I1 using, for examples, antibodies, in samples, like biological fluids and cells/ tissue samples .

5. A method for detecting increased tumor cell motility in a cancer patient comprising detecting the activity and/or expression of IL4I1 in a sample obtained from said patient, and detecting increased tumor cell motility in said patient, if said expression or enzymatic activity of said IL4I1 is increased in said patient when compared to control genes e.g, one or more housekeeping genes in said sample, or a control sample, e.g. a sample derived from a healthy subject, a group of healthy subjects, a prior sample from the same patient, a different patient or patient group, wherein preferably said cancer is **characterized by** a modulation, such as an increase of the activity and/or expression of AHR, and/or is preferably selected from the group consisting of B cell lymphoid malignancies, such as follicular lymphoma, Hodgkin lymphoma, primary mediastinal B cell lymphoma, diffuse large B cell lymphoma, marginal zone lymphoma and chronic lymphoid leukemia, ovarian carcinomas, mesotheliomas, colon carcinomas, breast carcinomas, melanomas, glioblastomas, prostate cancer, endometrial cancer, and lung carcinomas, preferably displaying IL4I1 - expressing cells.

6. A method for predicting or detecting the effect of cancer immunotherapy in a cancer patient comprising detecting the activity and/or expression of II,4I1 in a sample obtained from said patient, wherein said expression or enzymatic activity of said IL4I1 is increased in said patient when compared to control genes e.g, one or more housekeeping genes in said sample, or a control sample, e.g. a sample derived from a healthy subject, a group of healthy subjects, a prior sample from the same patient, a different patient or patient group is predictive for and/or indicates a reduced effect and/or immune evasion of said cancer immunotherapy in said patient, wherein preferably said increase of said activity and/or expression of said IL4I1 is detected in response to an immunotherapy in said patient.

7. A method for detecting resistance against a cancer treatment comprising immune therapy, for example immune checkpoint blockade (ICB), in a patient comprising detecting the activity and/or expression of IL4I1 in a sample obtained from said patient, and detecting resistance against a cancer treatment comprising immune therapy in said patient, if said expression or enzymatic activity of said IL4I1 is increased in said patient when compared to control genes e.g, one or more housekeeping genes in said sample, or a control sample, e.g. a sample derived from a healthy subject, a group of healthy subjects, a prior sample from the same patient, a different patient or patient group.

8. A method for predicting survival in a cancer patient comprising detecting the activity and/or expression of IL4I1 in a sample obtained from said patient, wherein said expression or enzymatic activity of said IL4I1 is increased in said patient when compared to control genes e.g, one or more housekeeping genes in said sample, or a control sample, e.g. a sample derived from a healthy subject, a group of healthy subjects, a prior sample from the same patient, a different patient or patient group is predictive for a reduced survival in said patient.

9. The method according to any one of claims 1 to 8, wherein said detecting of said activity comprises detecting IL4I1 metabolites, in particular IL4I1 tryptophan metabolites such as, for example I3P-derived metabolites, such as KynA, indole-3-acetic acid (IAA), indole-3-aldehyde (I3A) and/or indole-3-lactic acid (ILA) and indole-3-carbinol.

10. The method according to any one of claims 1 to 9, wherein said detecting of said activity comprises detecting the activity and/or expression of AHR, wherein an increase in said activity and/or expression of AHR when compared to a control is indicative for an increase of the activity of IL4I1.

11. The method according to any one of claims 1 to 10 wherein said biological sample is selected from a suitable sample comprising biological fluids, mammalian, for example human, cells, tissues, whole blood, cell lines, cellular supernatants, primary cells, IPSCs, hybridomas, recombinant cells, stem cells, and cancer cells, bone cells, cartilage cells, nerve cells, glial cells, epithelial cells, skin cells, scalp cells, lung cells, mucosal cells, muscle cells, skeletal muscles cells, striated muscle cells, smooth muscle cells, heart cells, secretory cells, adipose cells, blood cells, erythrocytes, basophils, eosinophils, monocytes, lymphocytes, T-cells, B-cells, neutrophils, NK cells, regulatory T-cells, dendritic cells, Th17 cells, Th1 cells, Th2 cells, myeloid cells, macrophages, monocyte derived stromal cells, bone marrow cells, spleen cells, thymus cells, pancreatic cells, oocytes, sperm, kidney cells, fibroblasts, intestinal cells, cells of the female or male reproductive tracts, prostate cells, bladder cells, eye cells, corneal cells, retinal cells, sensory cells, keratinocytes, hepatic cells, brain cells, kidney cells, and colon cells, and the transformed counterparts of said cells or tissues, and in particular cancer cells that do not derive from the immune system.

12. The method according to any one of claims 1 to 11, wherein said control sample is selected for example from a sample from a healthy subject or group of healthy subjects, a prior sample from the same patient, or a different patient or a patient group.

13. The method according to any one of claims 1 to 12, wherein said cancer is **characterized by** a modulation, such as an increase of the activity and/or expression of AHR, and/or is preferably selected from the group consisting of B cell lymphoid malignancies, such as follicular lymphoma, Hodgkin lymphoma, primary mediastinal B cell lymphoma, diffuse large B cell lymphoma, marginal zone lymphoma and chronic lymphoid leukemia, ovarian carcinomas, mesotheliomas, colon carcinomas, breast carcinomas, melanomas, glioblastomas prostate cancer, endometrial cancer, and lung carcinomas, preferably displaying IL4I1 -expressing cells.

14. The method according to any one of claims 1 to 13, further comprising the step of a stratification of said patient to a disease and/or treatment group, for example a treatment group receiving suitable IL4I1 inhibitors.

15. A diagnostic kit comprising materials for performing a method according to any one of claims 1 to 14 in one or separate containers, optionally together with auxiliary agents and/or instructions for performing said method.


SEQUENCE LISTING

<110>  Deutsches Krebsforschungszentrum Stiftung des öffentlichen
       Rechts
       Helmholtz-Zentrum für Umweltforschung GmbH - UFZ

<120>  Interleukin-4\226induced gene 1 (IL4I1) and respective metabolites
       as biomarkers for cancer

<130>  D31716EP

<160>  28

<170>  PatentIn version 3.5

<210>  1
<211>  97
<212>  DNA
<213>  Homo sapiens

<400>  1
tgctgttgac agtgagcgca ggaagaattg ttttaggata tagtgaagcc acagatgtat      60

atcctaaaac aattcttcct ttgcctactg cctcgga                               97


<210>  2
<211>  96
<212>  DNA
<213>  Homo sapiens

<400>  2
tgctgttgac agtgagcgaa ggcagaagta tgcaaagcat tagtgaagcc acagatgtaa      60

tgctttgcat acttctgcct gtgcctactg cctcgg                                96


<210>  3
<211>  21
<212>  DNA
<213>  Homo sapiens

<400>  3
ttccacgata tggatttacg g                                                21


<210>  4
<211>  21
<212>  DNA
<213>  Homo sapiens

<400>  4
gtttcctgtt gcattgagtc c                                                21


<210>  5
<211>  20
<212>  DNA
<213>  Homo sapiens

<400>  5
ccctcctcag gtggtgtttg                                                  20

<210> 6
<211> 20
<212> DNA
<213> Homo sapiens

<400> 6
cgacaaatga agcagcgtgt 20


<210> 7
<211> 21
<212> DNA
<213> Homo sapiens

<400> 7
gacgccttta tcctctctgc g 21


<210> 8
<211> 21
<212> DNA
<213> Homo sapiens

<400> 8
acgacctgat ccaattctgc c 21


<210> 9
<211> 21
<212> DNA
<213> Homo sapiens

<400> 9
ctgaccgcag agtcttttcc t 21


<210> 10
<211> 20
<212> DNA
<213> Homo sapiens

<400> 10
gagtggtttg gctggggtaa 20


<210> 11
<211> 21
<212> DNA
<213> Homo sapiens

<400> 11
ctgcctgggt ttccatcttc t 21


<210> 12
<211> 23
<212> DNA
<213> Homo sapiens

<400> 12
gccattcatg tcagagctac act 23

<210>  13
<211>  21
<212>  DNA
<213>  Homo sapiens

<400>  13
ctcgccagtg aaatgatggc t                                                 21


<210>  14
<211>  21
<212>  DNA
<213>  Homo sapiens

<400>  14
gtcggagatt cgtagctgga t                                                 21


<210>  15
<211>  20
<212>  DNA
<213>  Homo sapiens

<400>  15
cgcccgaaga catctaccag                                                   20


<210>  16
<211>  21
<212>  DNA
<213>  Homo sapiens

<400>  16
gatattccaa gagcgtgtgc c                                                 21


<210>  17
<211>  26
<212>  DNA
<213>  Homo sapiens

<400>  17
gctaaccttt gatgctataa ctacga                                            26


<210>  18
<211>  21
<212>  DNA
<213>  Homo sapiens

<400>  18
tttgtgcgca tgtagaatct g                                                 21


<210>  19
<211>  22
<212>  DNA
<213>  Homo sapiens

<400>  19
catcaatgac aaggtggcca ag                                                22

<210> 20
<211> 20
<212> DNA
<213> Homo sapiens

<400> 20
gggcttgatg gggtgatagg 20


<210> 21
<211> 19
<212> DNA
<213> Homo sapiens

<400> 21
gatgggcggc ggaaaatag 19


<210> 22
<211> 21
<212> DNA
<213> Homo sapiens

<400> 22
gcgtggattc tgcataatgg t 21


<210> 23
<211> 20
<212> DNA
<213> Homo sapiens

<400> 23
acccccatga ctccagagaa 20


<210> 24
<211> 21
<212> DNA
<213> Homo sapiens

<400> 24
cttgtgcctg caaaatcgca t 21


<210> 25
<211> 22
<212> DNA
<213> Homo sapiens

<400> 25
caccctctag caatgtcaac tc 22


<210> 26
<211> 21
<212> DNA
<213> Homo sapiens

<400> 26
cagactcggg atactctctc c 21

<210>  27
<211>  20
<212>  DNA
<213>  Homo sapiens

<400>  27
ggagcgcacc atcttcttca                                                    20


<210>  28
<211>  20
<212>  DNA
<213>  Homo sapiens

<400>  28
gggccgcatc ttcacctacc                                                    20



5